# EUROPEAN PATENT SPECIFICATION

(11) **EP 4 399 720 B1**
(45) Date of publication and mention of the grant of the patent: **24.12.2025**
(21) Application number: 22806089.3
(22) Date of filing: 07.09.2022
(51) Int. Cl.: G16H 50/00, G16H 70/40, G16B 40/00

(54) **CAUSAL NETWORK FOR DRUG DISCOVERY**
KAUSALES NETZWERK ZUR ARZNEIMITTELENTDECKUNG
RÉSEAU CAUSAL POUR LA DÉCOUVERTE DE MÉDICAMENTS

(30) Priority: 08.09.2021 US 202163241921 P
(43) Date of publication of application: 17.07.2024
(73) Proprietor: Enveda Therapeutics, Inc., Boulder, CO 80301 (US)
(72) Inventor: DOMINGO-FERNANDEZ, Daniel, Boulder, CO 80301 (US)
(74) Representative: J A Kemp LLP
(86) International application number: PCT/US2022/076049
(87) International publication number: WO 2023/039428

(56) References cited:
- US-A1- 2014 288 910
- SHUKLA RAMMOHAN ET AL: "Signature-based approaches for informed drug repurposing: targeting CNS disorders", NEUROPSYCHOPHARMACOLOGY, vol. 46, no. 1, 30 June 2020 (2020-06-30), pages 116 - 130, XP037305485, ISSN: 0893-133X, DOI: 10.1038/S41386-020-0752-6
- MOHAMMADASIF EMON ET AL: "PS4DR: a multimodal workflow for identification and prioritization of drugs based on pathway signatures", BMC BIOINFORMATICS, BIOMED CENTRAL LTD, LONDON, UK, vol. 21, no. 1, 5 June 2020 (2020-06-05), pages 1 - 21, XP021277521, DOI: 10.1186/S12859-020-03568-5
- DOMINGO-FERNÁNDEZ DANIEL ET AL: "Causal reasoning over knowledge graphs leveraging drug-perturbed and disease-specific transcriptomic signatures for drug discovery", PLOS COMPUTATIONAL BIOLOGY, vol. 18, no. 2, 25 February 2022 (2022-02-25), pages e1009909, XP093006123, DOI: 10.1371/journal.pcbi.1009909

## Description

### TECHNICAL FIELD

The present disclosure is directed to systems and methods that include causal networks for aspects of biological study and drug discovery, including the identification of biological pathways, or components thereof, associated with a stimulus and/or condition, identification of a drug useful for treating and/or preventing a condition, drug target prioritization, and drug repurposing.

### BACKGROUND

Proteomes, the collection of polypeptide species expressed in a cell, tissue, or organism, are vast and form complicated networks providing, *inter alia,* the signaling and machinery necessary for biological functions. The recent increases in available data and computing power have allowed for the analysis of proteomes and interactomes (sets of molecular interactions). However, such studies are hampered by the use of non-context-specific data (or improper context-specific data), an incomplete understanding of a full proteome and/or full interactome (*e.g.,* the human proteome and interactome are yet to be fully cataloged), and biasing towards well-studied polypeptides and their relationships. US 2024/288910 Al teaches traversing graph modalities of a knowledge graph via relationship descriptors, and filtering based on biological criteria indicated by a node descriptor.

### BRIEF SUMMARY

In some aspects, provided is a method of identifying a biological pathway that connects a stimulus and a condition in an individual, the method performed at a system comprising one or more processors, the method comprising: filtering a stimulus-condition knowledge graph using stimulus signature data to obtain a stimulus-filtered knowledge graph, wherein the stimulus-condition knowledge graph comprises: (a) nodes, each node representing the stimulus, biological entity, or the condition; and (b) causal edges, each causal edge representing a causal relationship connecting two nodes, wherein the stimulus signature data comprises information pertaining to biological entities associated with the stimulus, and wherein the stimulus-filtered knowledge graph comprises one or more qualifying paths according to the stimulus signature data; filtering the stimulus-filtered knowledge graph using condition signature data to obtain a condition-filtered knowledge graph to identify the biological pathway that connects the stimulus and the condition, wherein the condition signature data comprises information pertaining to biological entities associated with the condition, and wherein the condition-filtered knowledge graph comprises one or more qualifying paths according to the condition signature data.

In other aspects, provided is a method of identifying a biological pathway that connects a stimulus and a condition in an individual, the method performed at a system comprising one or more processors, the method comprising: filtering a stimulus-condition knowledge graph using condition signature data to obtain a condition-filtered knowledge graph, wherein the stimulus-condition knowledge graph comprises: (a) nodes, each node representing the stimulus, biological entities, or the condition; and (b) causal edges, each causal edge representing a causal relationship connecting two nodes, wherein the condition signature data comprises information pertaining to biological entities associated with the condition, and wherein the condition-filtered knowledge graph comprises one or more qualifying paths according to the condition signature data; and filtering the condition-filtered knowledge graph using stimulus signature data to obtain a stimulus-filtered knowledge graph to identify the biological pathway that connects the stimulus and the condition, wherein the stimulus signature data comprises information pertaining to biological entities associated with the stimulus, and wherein the stimulus-filtered knowledge graph comprises one or more qualifying paths according to the stimulus signature data.

In some embodiments, the method further comprises obtaining the stimulus-condition knowledge graph.

In some embodiments, the stimulus-condition knowledge graph is obtained from a master knowledge graph. In some embodiments, the master knowledge graph comprises information obtained from a database, text mining, and/ or proprietary source. In some embodiments, the master knowledge graph contains paths of length 20 or fewer. In some embodiments, obtaining the stimulus-condition knowledge graph from the master knowledge graph comprises reasoning over the master knowledge graph using a stimulus input representing the stimulus and a condition input representing the condition. In some embodiments, the method further comprises receiving the stimulus input and the condition input. In some embodiments, one or more paths connecting the stimulus and the condition of the stimulus-condition knowledge graph each have a length of 10 or fewer causal edges. In some embodiments, at least one of the one or more paths comprise a linear path. In some embodiments, at least one the one or more paths comprise a cyclical path. In some embodiments, the cyclical path is converted to a linear path. In some embodiments, the method further comprises generating and storing the stimulus-condition knowledge graph.

In some embodiments, the method further comprises displaying a visual representation comprising the stimulus-condition knowledge graph. In some embodiments, the visual representation of the stimulus-condition knowledge graph is overlaid with the master knowledge graph.

In some embodiments, the method comprises generating and storing the stimulus-filtered knowledge graph.

In some embodiments, the method further comprises generating and storing the condition-filtered knowledge graph.

In some embodiments, the causal relationship represented by the causal edge is based on a stimulus-biological entity interaction, a biological entity-biological entity interaction, or a biological entity-condition interaction. In some embodiments, the causal relationship of the stimulus-biological entity interaction represents an activation of the biological entity by the stimulus. In some embodiments, the causal relationship of the stimulus-biological entity interaction represents an inhibition of the biological entity by the stimulus. In some embodiments, the causal relationship of the biological entity-biological entity interaction represents an activation of a first biological entity by a second biological entity. In some embodiments, the causal relationship of the biological entity-biological entity interaction represents an inhibition of a first biological entity by a second biological entity. In some embodiments, the causal relationship of the biological entity-condition interaction represents a promotion of a condition by a biological entity. In some embodiments, the causal relationship of the biological entity-condition interaction represents a reversion of a condition by a biological entity.

In some embodiments, the method further comprises displaying a visual representation of the master knowledge graph.

In some embodiments, the stimulus signature data comprises up-regulation and/or down-regulation information for each biological entity of the stimulus signature data in the presence of the stimulus. In some embodiments, the stimulus signature data comprises information obtained from a database or proprietary source. In some embodiments, the stimulus signature data comprises information obtained from a stimulus-perturbation experiment. In some embodiments, the stimulus signature data comprises transcriptomic information.

In some embodiments, the method further comprises generating and displaying a visual representation comprising the stimulus-filtered knowledge graph. In some embodiments, the visual representation of the stimulus-filtered knowledge graph is overlaid with the stimulus-condition knowledge graph.

In some embodiments, the condition signature data comprises up-regulation and/or down-regulation information for each biological entity of the condition signature data due to the condition. In some embodiments, the condition signature data comprises information obtained from a database or proprietary source. In some embodiments, the condition signature data comprises information obtained from a condition-perturbation experiment. In some embodiments, the condition signature data comprises transcriptomic information.

In some embodiments, the method further comprises displaying a visual representation comprising the condition-filtered knowledge graph. In some embodiments, the visual representation of the condition-filtered knowledge graph is overlaid with the stimulus-condition knowledge graph.

In some embodiments, the one or more qualifying paths comprises a set of causal edges in complete agreement with the stimulus signature data and/ or condition signature data. In some embodiments, the one or more qualifying paths comprises a set of causal edges comprising one error that is not in agreement with the stimulus signature data and/ or condition signature data.

In some embodiments, the method further comprises scoring paths of the identified biological pathway connecting the stimulus and the condition.

In some embodiments, the method further comprises determining a target biological entity based on the identified biological pathway for further assessment.

In some embodiments, the stimulus is a drug or an environmental stimulus. In some embodiments, the stimulus is a plant extract. In some embodiments, the condition is a disease. In some embodiments, the biological entity is a polypeptide.

In some embodiments, the individual is a human.

### BRIEF DESCRIPTION OF THE DRAWINGS

**FIG. 1** shows a schematic of a computing device useful for the methods described herein.
**FIG. 2** shows a schematic of an example workflow **100** of a method, and aspects thereof, for identifying a biological pathway according to the description herein.
**FIG. 3** shows a schematic of an example workflow **200** of a method, and aspects thereof, for identifying a drug-disease pair according to the description herein.
**FIG. 4** shows a schematic of steps of a workflow **300** of a method, and aspects thereof, for identifying a biological pathway connecting a drug and a disease, and further drug prioritization.
**FIG. 5** shows visualizations of the distributions of nodes and edges in custom KG and subsections thereof.
**FIG. 6** shows the distribution of nodes and edges in the custom KG and OpenBioLink KG.
**FIG. 7** shows a schematic of an example workflow **700** of a method, and aspects thereof, for drug repurposing.

### DETAILED DESCRIPTION

Provided herein, in certain aspects, are methods of developing and using causal networks for various aspects of biological study and drug discovery. For example, in certain embodiments, provided are methods of identifying a biological pathway, or component thereof, that connects a stimulus and a condition in an individual. In some embodiments, the stimulus is a medical intervention, such as a drug or plant extract (or components thereof), and the condition is a disease, such as a cancer. In some embodiments, the stimulus is an environmental or personal-choice exposure, such as a smoke-based toxin, and the condition is a disease. In some embodiments, the condition is a side effect, such as a side effect of a drug. These causal networks can then be used to, for example, identify a drug useful for treating a disease, such as from a plant extract, drug repurposing, and identification of drug mechanism (including the cause of a side effect) or druggable target.

The present application is based, at least in part, on the inventors' unexpected findings associated with a computer-implementable approach for identifying and prioritizing biological pathways by assessing causal paths in a knowledge graph (KG) format using stimulus-perturbed and condition-perturbed data. The techniques taught herein integrate signature data into the overall methodology to provide powerful and robust causal network analyses that enable, *e.g.,* high confidence identification of a biological pathway connecting a stimulus and condition. The described approaches demonstrate an ability to correctly classify a large proportion of known and clinically investigated drug-disease pairs. Furthermore, such approaches demonstrate an ability to perform hypothesis generation (such as proposed mechanisms of action for a drug) and drug target prioritization.

Thus, in some aspects, provided herein is a method of identifying a biological pathway that connects a stimulus and a condition in an individual, the method performed at a system comprising one or more processors, the method comprising: filtering a stimulus-condition knowledge graph using stimulus signature data to obtain a stimulus-filtered knowledge graph, wherein the stimulus-condition knowledge graph comprises: (a) nodes, each node representing the stimulus, biological entity, or the condition; and (b) causal edges, each causal edge representing a causal relationship connecting two nodes, wherein the stimulus signature data comprises information pertaining to biological entities associated with the stimulus, and wherein the stimulus-filtered knowledge graph comprises one or more qualifying paths according to the stimulus signature data; filtering the stimulus-filtered knowledge graph using condition signature data to obtain a condition-filtered knowledge graph to identify the biological pathway that connects the stimulus and the condition, wherein the condition signature data comprises information pertaining to biological entities associated with the condition, and wherein the condition-filtered knowledge graph comprises one or more qualifying paths according to the condition signature data. In some embodiments, the stimulus is a drug or plant extract. In some embodiments, the condition is a condition of an individual, such as an experienced side effect, symptom, or disease.

In other aspects, provided is a method of identifying a biological pathway that connects a stimulus and a condition in an individual, the method performed at a system comprising one or more processors, the method comprising: filtering a stimulus-condition knowledge graph using condition signature data to obtain a condition-filtered knowledge graph, wherein the stimulus-condition knowledge graph comprises: (a) nodes, each node representing the stimulus, biological entities, or the condition; and (b) causal edges, each causal edge representing a causal relationship connecting two nodes, wherein the condition signature data comprises information pertaining to biological entities associated with the condition, and wherein the condition-filtered knowledge graph comprises one or more qualifying paths according to the condition signature data; and filtering the condition-filtered knowledge graph using stimulus signature data to obtain a stimulus-filtered knowledge graph to identify the biological pathway that connects the stimulus and the condition, wherein the stimulus signature data comprises information pertaining to biological entities associated with the stimulus, and wherein the stimulus-filtered knowledge graph comprises one or more qualifying paths according to the stimulus signature data. In some embodiments, the stimulus is a drug or plant extract. In some embodiments, the condition is a condition of an individual, such as an experienced side effect, symptom, or disease.

### I. Definitions

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The terms "polypeptide" and "protein," as used herein, may be used interchangeably to refer to a polymer comprising amino acid residues, and are not limited to a minimum length. Such polymers may contain natural or non-natural amino acid residues, or combinations thereof, and include, but are not limited to, peptides, polypeptides, oligopeptides, dimers, trimers, and multimers of amino acid residues. Full-length polypeptides or proteins, and fragments thereof, are encompassed by this definition. The terms also include modified species thereof, *e.g.,* post-translational modifications of one or more residues, for example, methylation, phosphorylation glycosylation, sialylation, or acetylation.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For instance, where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both limits, ranges excluding either or both of those included limits are also included in the disclosure. In some embodiments, two opposing and open-ended ranges are provided for a feature, and in such description it is envisioned that combinations of those two ranges are provided herein. For example, in some embodiments, it is described that a feature is greater than about 10 units, and it is described (such as in another sentence) that the feature is less than about 20 units, and thus, the range of about 10 units to about 20 units is described herein.

The term "about" as used herein refers to the usual error range for the respective value readily known in this technical field. Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise. For example, "a" or "an" means "at least one" or "one or more." It is understood that aspects and variations described herein include embodiments "consisting" and/or "consisting essentially of" such aspects and variations.

As used herein, a "subject" or an "individual," which are terms that are used interchangeably, is a mammal. In some embodiments, a "mammal" includes humans, non-human primates, domestic and farm animals, and zoo, sports, or pet animals, such as dogs, horses, rabbits, cattle, pigs, hamsters, gerbils, mice, ferrets, rats, cats, monkeys, *etc.* In some embodiments, the subject or individual is human.

Those skilled in the art will recognize that several embodiments are possible within the scope of the present disclosure. The following description illustrates the disclosure and, of course, should not be construed in any way as limiting the scope of the inventions described herein, which is defined by the appended claims.

### II. Methods

Provided herein are methods of developing and using causal networks for various aspects of biological study and drug discovery. In certain aspects, the method described herein comprise filtering pathway predictions formed from known literature information using signature data, such as a stimulus signature data and/or a condition signature data, *e.g.,* obtained from context-specific perturbation studies. The integration of signature data provides powerful and robust techniques for causal network analysis enabling, *e*.*g*., confident identification of a biological pathway connecting a stimulus and condition.

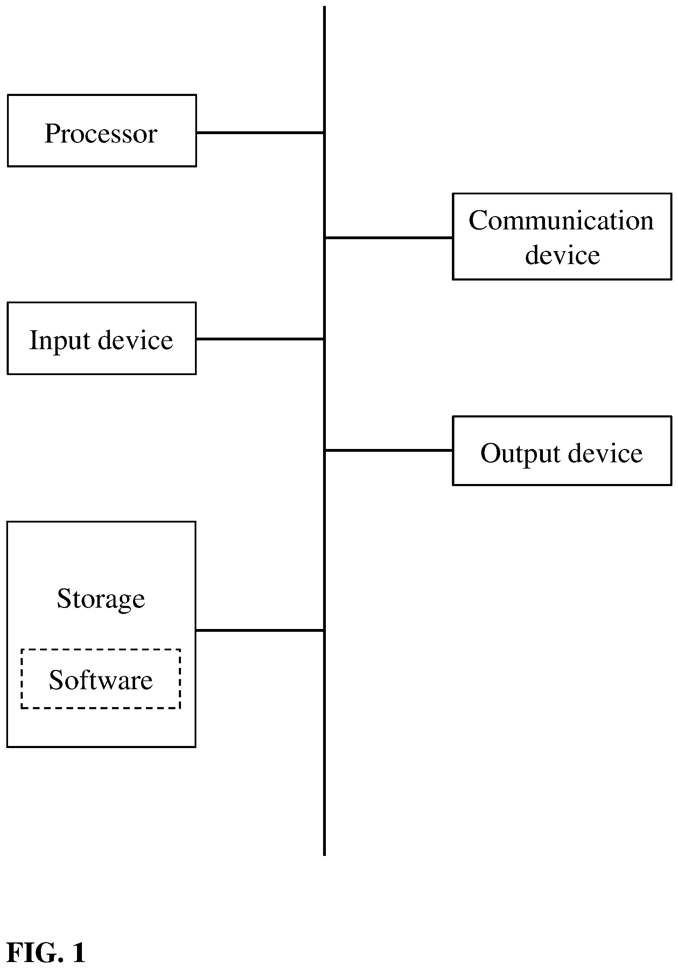

The methods described herein, in certain aspects, are implemented via a device, such as a computer. As shown in **FIG. 1****,** in some embodiments, the device is a host computer connected to a network. In some embodiments, the device is a client computer or a server. In some embodiments, the device can be any suitable type of microprocessor-based device, such as a personal computer, workstation, server or handheld computing device (portable electronic device) such as a phone or tablet. The device can include, for example, one or more of processor, input device, output device, storage, and communication device. The input device and output device can generally correspond to those described above, and can either be connectable or integrated with the device. For example, the input device can be any suitable device that provides input, such as a touch screen, keyboard or keypad, mouse, or voice-recognition device. The output device can be any suitable device that provides output, such as a touch screen, haptics device, or speaker. The storage can be any suitable device that provides storage, such as an electrical, magnetic or optical memory including a RAM, cache, hard drive, or removable storage disk. The communication device can include any suitable device capable of transmitting and receiving signals over a network, such as a network interface chip or device. The components of the computer can be connected in any suitable manner, such as via a physical bus or wirelessly. The software, which can be stored in the storage and executed by the processor, can include, for example, the programming that embodies the functionality of the present disclosure (e.g., as embodied in the devices as described above). The software can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as the storage, that can contain or store programming for use by or in connection with an instruction execution system, apparatus, or device. The software can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport readable medium can include, but is not limited to, an electronic, magnetic, optical, electromagnetic, or infrared wired or wireless propagation medium. The device may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines. The device can implement any operating system suitable for operating on the network. The software can be written in any suitable programming language, such as C, C++, Java or Python. In various embodiments, application software embodying the functionality of the present disclosure can be deployed in different configurations, such as in a client/server arrangement or through a Web browser as a Web-based application or Web service, for example.

In some embodiments, the storage is a non-transitory computer-readable storage medium storing one or more programs, the one or more programs comprising instructions, which when executed by one or more processors of an electronic device, cause the electronic device to complete a method described herein, or an aspect thereof.

In some embodiments, provided is a method comprising storing a knowledge graph (KG) database on a device, such as a computer. In some embodiments, the KG database format comprises database nodes containing the information contained in a KG (such as described in more detail herein). The KG database format allows for information contained in a KG, such as stimulus nodes, biological entity nodes, condition nodes, and causal edges to be more quickly and efficiently accessed and analyzed on the device, such as the computer.

In some embodiments, the knowledge graphs described herein are created and stored using known software packages that are configured for creating and storing KG databases. For example, the knowledge graphs described herein can be created and stored using KG library software, such as Neo4j, Biological Expression Language (BEL), OrientDB, MongoDB, or ArangoDB. In some embodiments, the method comprises filtering the KG database using NetworkX, NetworkKit, igraph, enaR, ggnet2, or pajek.

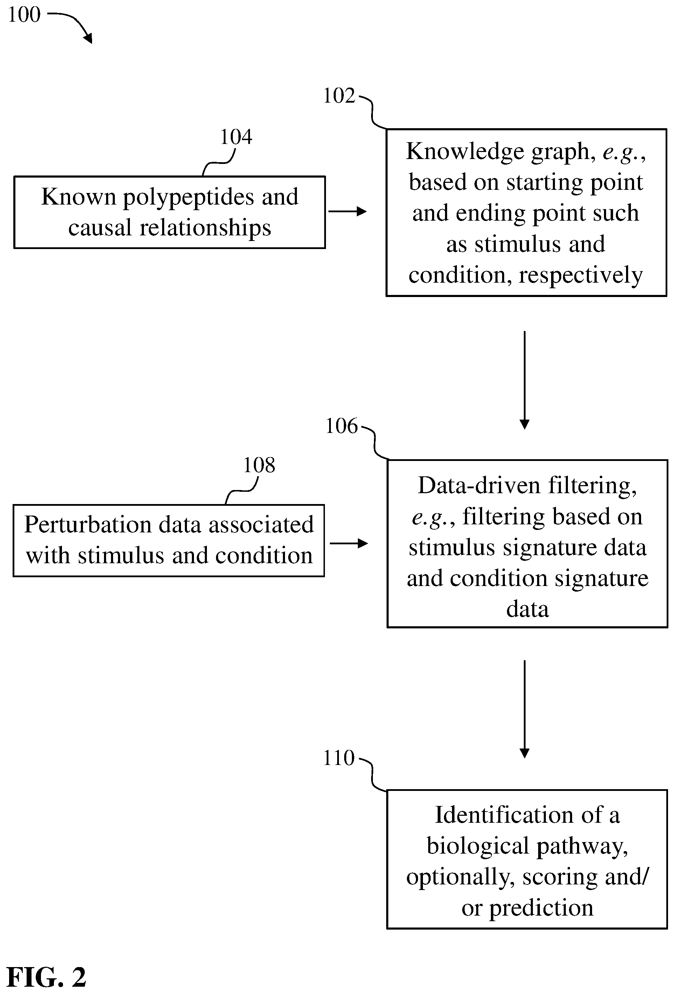

For purposes of describing and illustrating the methods taught herein, a schematic of an example workflow **100** of a method, and aspects thereof, for identifying a biological pathway is provided in **FIG. 2****.** In some embodiments, the method begins with a knowledge graph (KG) **102** containing information regarding a starting point (such as a stimulus), an ending point (such as a condition), known biological entities (such as polypeptides), and causal relationships that are predicted to connect the starting point and ending point. The KG can be constructed from known polypeptides and causal relationships therebetween and with the stimulus and condition **104.** In some embodiments, the information used to construct the KG is obtained from literature sources. As illustrated, the workflow **100** involves data-driven filtering **106** of the KG, such as using stimulus signature data and/or condition signature data. In some embodiments, the methods comprise sequential filtering steps, *e.g.,* a first filtering step using stimulus signature data and a second filtering step using condition signature data. In some embodiments, the data used for filtering is obtained from context-specific perturbation studies **108,** such as experimental perturbation studies, and may reflect change in one or more polypeptide due to perturbation by the stimulus or condition. For example, in some embodiments, the signature data reflects changes in polypeptide expression (such as obtained from transcriptomic data) due to the presence of the stimulus or the condition. Filtering based on signature data **106** enables the identification of biological pathways **110** that connect the starting point and the condition, such as the stimulus and condition, via one or more paths. In some embodiments, the identified paths of a biological pathway are scored to enable further prediction and/ or ranking of aspects of the identified biological pathways **110.**

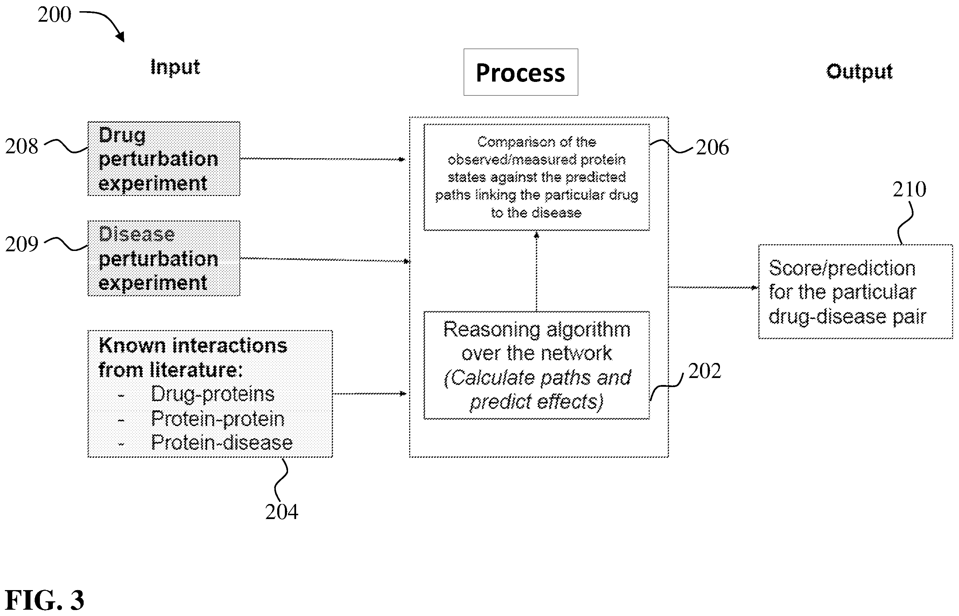

**FIG. 3** provides a schematic of an example workflow **200** of a method, and aspects thereof, for identifying a biological pathway associated with a drug-disease pair, and further scoring and prediction of aspects of the identified biological pathway. A reasoning algorithm is used to generate a KG **202** using known interactions from the literature **204,** such as from known drug-polypeptide, polypeptide-polypeptide, and polypeptide-disease information. The reasoning algorithm calculates paths and predicts effects of a drug and how that may be associated with a disease via the known interactions. Next, a comparison of observed/ measured polypeptide states (such as expression data from transcriptomic experiments) are performed against the predicted paths of the KG linking the drug and the disease **206.** Specifically, the comparison is based on signature data from a drug perturbation experiment **208** and a disease perturbation experiment **209.** Biological pathways connecting the drug and disease are identified via the comparison step(s), and further scoring and prediction can be performed **210** to assess the drug-disease pair and paths connecting the drug and disease. For example, in some embodiments, a biological pathway connecting a drug and a disease may have many paths, and scoring can help to evaluate and/or prioritize the paths and/or component thereof.

In some aspects, provided is a method of identifying a biological pathway that connects a stimulus and a condition in an individual, the method performed at a system comprising one or more processors, the method comprising: filtering a stimulus-condition knowledge graph using stimulus signature data to obtain a stimulus-filtered knowledge graph, wherein the stimulus-condition knowledge graph comprises: (a) nodes, each node representing the stimulus, biological entity, or the condition; and (b) causal edges, each causal edge representing a causal relationship connecting two nodes, wherein the stimulus signature data comprises information pertaining to biological entities associated with the stimulus, and wherein the stimulus-filtered knowledge graph comprises one or more qualifying paths according to the stimulus signature data; filtering the stimulus-filtered knowledge graph using condition signature data to obtain a condition-filtered knowledge graph to identify the biological pathway that connects the stimulus and the condition, wherein the condition signature data comprises information pertaining to biological entities associated with the condition, and wherein the condition-filtered knowledge graph comprises one or more qualifying paths according to the condition signature data.

In other aspects, provided is a method of identifying a biological pathway that connects a stimulus and a condition in an individual, the method performed at a system comprising one or more processors, the method comprising: filtering a stimulus-condition knowledge graph using condition signature data to obtain a condition-filtered knowledge graph, wherein the stimulus-condition knowledge graph comprises: (a) nodes, each node representing the stimulus, biological entities, or the condition; and (b) causal edges, each causal edge representing a causal relationship connecting two nodes, wherein the condition signature data comprises information pertaining to biological entities associated with the condition, and wherein the condition-filtered knowledge graph comprises one or more qualifying paths according to the condition signature data; and filtering the condition-filtered knowledge graph using stimulus signature data to obtain a stimulus-filtered knowledge graph to identify the biological pathway that connects the stimulus and the condition, wherein the stimulus signature data comprises information pertaining to biological entities associated with the stimulus, and wherein the stimulus-filtered knowledge graph comprises one or more qualifying paths according to the stimulus signature data.

In the following sections, additional description of the various steps and method steps is provided. Such description in a modular fashion is not intended to limit the scope of the disclosure and based on the teachings provided herein one of ordinary skill in the art will readily appreciate that certain modules can be integrated, at least in part. The section heading used herein are for organizational purposes only and are not to be construed as limiting the subject matter described.

### A. Knowledge graphs

In certain aspects, the methods, and associated steps, provided herein involve a knowledge graph (KG) database format. KGs contain a broad range of biological information and scales, including from the genetic and molecular levels (*e.g*., polypeptides, drugs, and biochemicals) to biological concepts (*e.g.,* phenotypes and disease). In some embodiments, the KG comprises (a) nodes, each node representing a stimulus, a biological entity, or a condition; and (b) causal edges, each causal edge representing a causal relationship connecting two nodes. In some embodiments, the KG comprises a biological pathway (such as a predicted biological pathway) connecting a stimulus and a condition, wherein the biological pathway comprises a plurality of paths. Nodes may contain various pieces of information regarding the stimulus, the biological entity, or the condition. In some embodiments, the node comprises identity information, such as a name of a drug, polypeptide, or condition. In some embodiments, the node comprises additional information, such as a level of the entity represented by the node, *e.g.,* whether a polypeptide is overexpressed or underexpressed. In some embodiments, the KG comprises one or more, such as at least any of 5, 10, 50, 100, stimuli nodes. In some embodiments, the KG comprises one or more, such as at least any of 5, 10, 50, or 100, condition nodes.

As discussed herein, in some embodiments, the node represents a stimulus, such as a drug or plant extract (in certain aspects, such a node is also referred to herein as a stimulus node). In some embodiments, the node represents a condition, such as a symptom, side effect, or a disease (in certain aspects, such a node is also referred to herein as a condition node). More details regarding stimuli and conditions are provided in a later section.

In some embodiments, the node represents a biological entity (in certain aspects, such a node is also referred to herein as a biological entity node). The KGs encompassed by the description provided herein may include biological entity nodes encompassing information regarding a diverse array biological molecules or biological components and observable characteristics associated with a stimulus and/or condition, including via a pathway between a stimulus and condition. In some embodiments, the biological entity node represents a polypeptide, such as a protein. In some embodiments, the biological entity node represents a nucleic acid, such as a DNA or RNA molecule. In some embodiments, the biological entity node represents a biological complex, such as a complex of polypeptides and/ or nucleic acids. In some embodiments, the biological entity node represents a metabolite. In some embodiments, the biological entity node represents an observable characteristic of a biological entity, such as a morphological characteristic of a cell or cell cycle characteristic. In some embodiments, the biological entity node represents a state of inflammation.

In some embodiments, the biological entity node represents a polypeptide, including a polypeptide species, *e.g.,* a polypeptide having a specific post-translational modification (such as a post-translation modification of one or more of methylation, phosphorylation glycosylation, sialylation, and acetylation), a polypeptide isoform, or a polypeptide variant (such as a spliced variant). In some embodiments, the polypeptide and associated species, such as species having a post-translation modification and/or an isoform thereof, are represented in a single node. In some embodiments, the polypeptide and associated species, such species having a post-translation modification and/or an isoform thereof, are represented in more than one node. In some embodiments, each species of a polypeptide, such species having a post-translation modification and/or an isoform thereof, is represented in a separate node.

As biological entity nodes may encompass a diverse array of biological molecules or biological components and observable characteristics, KGs encompassed by the description provided herein may include biological entity nodes focused on a certain type of biological entity or any mixture thereof. For example, in some embodiments, the KG comprises biological entity nodes only pertaining to polypeptides and species thereof.

The causal edges described herein are based on information regarding a known or predicted relationship between two nodes (in certain aspects, such a relationship is referred to herein as a causal relationship), and include information regarding the impact of an entity represented by a first node on another entity represented by a second node. For example, in some embodiments, the causal edge provides information regarding a stimulus-biological entity relationship (*i.e.,* the causal relationship between a stimulus node and a biological entity node). In some embodiments, the causal edge provides information regarding a biological entity-biological entity relationship (*i.e.,* the causal relationship between a first biological entity node and a second biological entity node). In some embodiments, the causal edge provides information regarding a biological entity-condition relationship (*i.e.,* the causal relationship between a biological entity node and a condition node).

In some embodiments, the causal relationship represented by a causal edge is directional (*e.g.,* a first biological entity represented by one node activates or inhibits a second biological entity represented by another node). In some embodiments, the causal relationship represented by the causal edge is based on a stimulus-biological entity interaction, a biological entity-biological entity interaction, or a biological entity-condition interaction. In some embodiments, the causal relationship of the stimulus-biological entity interaction represents an activation of the biological entity by the stimulus. In some embodiments, the causal relationship of the stimulus-biological entity interaction represents an inhibition of the biological entity by the stimulus. In some embodiments, the causal relationship of the biological entity-biological entity interaction represents an activation of a first biological entity by a second biological entity. In some embodiments, the causal relationship of the biological entity-biological entity interaction represents an inhibition of a first biological entity by a second biological entity. In some embodiments, the causal relationship of the biological entity-condition interaction represents a promotion of a condition by a biological entity. In some embodiments, the causal relationship of the biological entity-condition interaction represents a reversion of a condition by a biological entity.

In certain aspects, the causal edge implies a direction of regulation, *e.g.,* a first node upregulates or downregulates a second node. In some embodiments, as pertaining to causal relationships involving biological entities, activation is based on an increase in the amount and/or one or more functions of the biological entity. In some embodiments, as pertaining to causal relationships involving biological entities, inhibition is based on a decrease in the amount and/or one or more functions of the biological entity. In some embodiments, the amount of a biological entity is assessed via a gene expression product associated with the biological entity, *e.g.,* when the biological entity node represents a polypeptide, the amount is assessed via RNA expression, such as obtained from transcriptomic data, and/or polypeptide expression, such as obtained from mass spectrometry data. In some embodiments, the one or more functions of a biological entity are assessed via a functional assay, such as a signaling or enzymatic assay.

In some embodiments, as pertaining to causal relationships involving conditions, promotion is based on an increase in a feature associated with the condition, such as initiation, progression, or recurrence of the condition, the presence or increased severity of one or more symptoms associated with the condition, and the need for increased treatment and/or prevention to mitigate the condition and/or maintain a certain quality of life. In some embodiments, as pertaining to causal relationships involving conditions, reversion is based on a decrease in a feature associated with the condition, such as remission or curing of the condition, the disappearance or reduced severity of one or more symptoms associated with the condition, and the ability to reduce treatment and/or prevention to mitigate the condition and/or maintain a certain quality of life.

In some embodiments, the KG comprises one or more paths connecting a stimulus and a condition. As described herein, a path is a series of nodes connected by causal edges that links and include a stimulus node and a condition node. In the method described herein, paths may be described in a number of ways based on the composition of the nodes and causal edges constituting the path. In some embodiments, the path comprises a plurality of nodes, wherein each node of the path is different (*e.g.,* each biological entity node of the path is different). In some embodiments, the path comprises a plurality of nodes, wherein the plurality of nodes comprises two or more identical nodes (*e.g.,* the path has two biological entity nodes representing the same biological entity). In some embodiments, the path is a linear path. In some embodiments, the path is a cyclic path, *e.g.,* a path comprising a repeating node that forms a loop. In some embodiments, the path is an acyclic path. In some embodiments, the cyclic path is converted to one or more acyclic paths representing the possible paths of the cyclic path. In certain aspects, the KG comprises a single version of a path that may be represented in two or more equivalent manners. In some embodiments, the KG comprises only acyclic paths, including linear paths.

As described herein, in some embodiments, the path may be characterized by an attribute thereof, such as length based on the number of nodes and/or the number of causal edges. In some embodiments, the length of a path is described by the number of causal edges. Generally, the KGs described herein may have paths of any length, and, in some embodiments, it may be desirable to limit the KG to paths having a maximum length. In some embodiments, the KG comprises paths of 20 or fewer causal edges, such as any of 19 or fewer causal edges, 18 or fewer causal edges, 17 or fewer causal edges, 16 or fewer causal edges, 15 or fewer causal edges, 14 or fewer causal edges, 13 or fewer causal edges, 12 or fewer causal edges, 11 or fewer causal edges, 10 or fewer causal edges, 9 or fewer causal edges, 8 or fewer causal edges, 7 or fewer causal edges, 6 or fewer causal edges, or 5 or fewer causal edges.

The information required to produce a KG may come from a variety of sources. In some embodiments, the KG comprises information obtained from a database, such as a publicly available database (*see, e.g.,* Riffle & Eng, Proteomics, 9, 2009, which is hereby incorporated by reference in its entirety). In some embodiments, the information obtained from the database is from one or more of Uniprot, Proteomics DB, PRIDE, ProteomeXchange, CCSB Interactome Database, IntAct Molecular Interaction Database, PubChem, Mondo Disease Ontology, ENTRAZ, STRING, DisGeNET, Hetionet, WikiPathways, and Reactome. In some embodiments, the KG comprises information obtained from text mining. In some embodiments, text mining is performed on scientific publications. In some embodiments, text mining is performed by identifying two nodes connected by a causal edge, *e.g.,* two polypeptides in proximity to a verb indicating activation or inhibition. Text mining techniques are well known in the art, *see, e.g.,* Sharp et al., ACL Anthology, 2019 (N19-4008); and Noriega-Atala et al., ACL Anthology, 2019 (W19-2603). In some embodiments, the KG comprises proprietary information, *e.g.,* based on compilations of verified information and/or experimental work (*e.g.,* transcriptomic and/or proteomic experiments). In some embodiments, the KG comprises information that is not context specific, *e.g.,* may not pertain to a specific stimulus and/or condition. In some embodiments, the KG comprises information that is context specific. In some embodiments, the KG comprises information having a desired level of confidence and/or quality, *e.g.,* a KG comprises causal edges measured in a disease-specific context.

In some embodiments, the information gathered to construct a KG may contain counter opposing information. For example, in some embodiments, information may indicate that a first biological entity activates a second biological entity, and information may indicate that the first biological entity inhibits the second biological entity. In some embodiments, such counter opposing information is excluded from a KG. In some embodiments, counter opposing information is assessed and a causal edge is included in a KG based on a weighting factor applied to the counter opposing information. For example, in some embodiments, counter opposing information may comprise 5 indications of a first directionality connecting two nodes and 1 indication of an opposing directionality to the two nodes, and in such instance the majority supported directionality is included in the KG. In some embodiments, such weighting is based on confidence of source information for determining the causal edge.

In some embodiments, the method provided here comprises a step and/or feature associated with the computer implementation of the method. For example, in some embodiments, the method comprises generating the KG. In some embodiments, the method comprises storing the KG. In some embodiments, the method comprises displaying a visual representation comprising the KG. In some embodiments, the visual representation of the KG is overlaid with another KG. In some embodiments, the visual representation of the KG is interactive, *e.g.,* a user may change views to view certain aspects of a KG or subsections of the KG.

In some embodiments, the methods described herein involve the use and/or production or more than one knowledge graph. In some embodiments, such as those described below, the naming of a KG is intended to provide a means for labeling and facilitating discussion of the KG, and is not intended to necessarily limit the contents and/or use of the KG. For example, a KG may be described herein using a descriptor of the last processing step, *e.g.,* a filtering step, applied thereto, and does not exclude the possibility of other prior processing steps having been applied to the KG or a precursor thereof.

### i. Master knowledge graphs

In some embodiments, the KG is a master KG. In some embodiments, the master KG comprises information regarding numerous stimuli (*e.g.,* drugs and/or environmental conditions and/or genetic status) and/or conditions (*e.g.,* disorders and/or diseases and/or phenotypes). In some embodiments, the master KG contains paths of any length (*e.g.,* paths containing any number of causal edges). In some embodiments, the master KG comprises information obtained from databases, text mining, and/or proprietary sources.

In some embodiments, the master KG comprises one or more, such as at least any of 5, 10, 50, 100, stimuli nodes. In some embodiments, the master KG comprises one or more, such as at least any of 5, 10, 50, or 100, condition nodes. In some embodiments, the master KG comprises, such as only contains, paths based on known biochemical pathways, such as the Wnt-signaling pathway. In some embodiments, the master KG comprises, such as only contains, context specific paths, such as paths with a known association to a disease, such as a cancer (including cancer subtype), or paths with a known association to a cell type.

In some embodiments, the paths of a master KG are all acyclic. In some embodiments, the KG comprises an acyclic path that is a representation of a cyclic path or a portion thereof. In some embodiments, the master KG does not comprise a cyclic path. In some embodiments, the paths in a master KG have a maximum length, as measured by the number of casual edges, of 20 or fewer causal edges, such as any of 19 or fewer causal edges, 18 or fewer causal edges, 17 or fewer causal edges, 16 or fewer causal edges, 15 or fewer causal edges, 14 or fewer causal edges, 13 or fewer causal edges, 12 or fewer causal edges, 11 or fewer causal edges, 10 or fewer causal edges, 9 or fewer causal edges, 8 or fewer causal edges, 7 or fewer causal edges, 6 or fewer causal edges, or 5 or fewer causal edges. In certain aspects, the application of a maximum path length in a KG database format allows for information contained in a KG, such as stimulus nodes, biological entity nodes, condition nodes, and causal edges to be more quickly and efficiently accessed and analyzed on the device, such as the computer.

In some embodiments, the method comprises obtaining (such as generating) a master KG. In some embodiments, the method comprises storing a master KG. In some embodiments, the method comprises obtaining at least a portion of the information in a master KG. In some embodiments, the method comprises displaying a visual representation of a master KG. In some embodiments, the visual representation of the master KG is interactive, *e.g.,* a user may change views to view certain aspects of a master KG or subsections of the master KG.

### ii. Stimulus-condition knowledge graphs

In some embodiments, the KG is a stimulus-condition KG. In some embodiments, the stimulus-condition KG is subset of a master KG, wherein the stimulus-condition KG comprises biological entity nodes and causal edges creating paths (a biological pathway) between one or more stimuli and one or more conditions. For example, the stimulus-condition KG comprises a stimulus node, a condition node, and causal edges and biological entity nodes connecting the stimulus node and the condition node. In some embodiments, the stimulus-condition KG comprises one or more, such as any of 2, 3, 4, or 5, stimuli nodes. In some embodiments, the stimulus-condition KG comprises one or more, such as any of 2, 3, 4, or 5, condition nodes.

In some embodiments, the method comprises reasoning over the master KG to obtain a stimulus-condition KG. In some embodiments, reasoning is based, at least in part, on selection (including input) of one or more stimulus nodes. In some embodiments, reasoning is based, at least in part, on selection (including input) of one or more condition nodes. In some embodiments, reasoning is based, at least in part, on identification of paths between a stimulus node and a condition node. In some embodiments, the paths of a stimulus-condition KG are all acyclic. In some embodiments, the stimulus-condition KG comprises an acyclic path that is a representation of a cyclic path or a portion thereof. In some embodiments, the stimulus-condition KG does not comprise a cyclic path. In some embodiments, paths connecting a stimulus and a condition are limited based on length. In some embodiments, the path length, as defined by the number of causal edges, of paths of a stimulus-condition KG is 10 or fewer causal edges, such as any of 9 or fewer causal edges, 8 or fewer causal edges, 7 or fewer causal edges, 6 or fewer causal edges, 5 or fewer causal edges, 4 or fewer causal edges, 3 or fewer causal edges, or 2 or fewer causal edges. In certain aspects, the application of a maximum path length in a KG database format allows for information contained in a KG, such as stimulus nodes, biological entity nodes, condition nodes, and causal edges to be more quickly and efficiently accessed and analyzed on the device, such as the computer.

In some embodiments, the method comprises obtaining (such as generating) a stimulus-condition KG. In some embodiments, the stimulus-condition KG is obtained from a master KG. In some embodiments, obtaining the stimulus-condition KG from the master KG comprises filtering the master KG using a stimulus input representing the stimulus and a condition input representing the condition. In some embodiments, the method comprises receiving the stimulus input and the condition input. In some embodiments, the method comprises storing a stimulus-condition KG. In some embodiments, the method comprises displaying a visual representation of a stimulus-condition KG. In some embodiments, the visual representation of the stimulus-condition KG is interactive, *e.g.,* a user may change views to view certain aspects of a stimulus-condition KG or subsections of the stimulus-condition KG. In some embodiments, the visual representation of the stimulus-condition KG is overlaid with a master KG.

### iii. Filtered knowledge graphs

As described herein, in certain aspects, the method comprises filtering a KG, such as a stimulus-condition KG, based on signature data. Thus, the methods encompass obtaining, such as generating or producing, filtered KGs, such as a stimulus-filtered KG or a condition-filtered KG. As described herein, the use of the terms stimulus-filtered KG and condition-filtered KG do not imply that this is the only filtering done to arrive at the stimulus-filtered KG or the condition-filtered KG. For example, in some embodiments, provided is a stimulus-filtered KG, which may or may not also reflect previous filtering based on condition signature data. In some embodiments, provided is a condition-filtered KG, which may or may not also reflect filtering based on stimulus signature data. In some embodiments, the filtered KG comprises only certain qualifying paths, such that the qualifying paths satisfy the applied requirements in view of signature data, *e.g.,* a stimulus-filtered KG comprises paths that fully correlate with the stimulus signature data based on the causal edges of a stimulus-condition KG *(i.e.,* all causal edges of the path are found to be concordant with the stimulus signature data). In some embodiments, the qualifying path allows for one or more errors.

As described in other sections, filtering is based on obtaining one or more paths where the signature data is in agreement, at least to a degree, with an implied result of the biological entity node(s) and condition node(s) based on a causal edge leading thereto. It is important to note that agreement is assessed in view of the signature data being applied to a KG. For example, in some embodiments, the path is in agreement with drug signature data when each causal edge of the path is concordant with the drug signature data. In some embodiments, the path is in agreement with disease signature data when the disease signature data is opposite (anti-correlate) of the drug signature data and/or the implied results of the biological entity node(s) and/or disease node(s) based on causal edges directional from a drug node to a disease node. Thus, the filtered KGs described herein comprise one or more paths that are not removed during the filtering process. In some embodiments, paths removed during the filtering process (and thus no longer included in downstream filtered KGs) are not in agreement, at least to a degree, with signature data.

In some embodiments, the paths of a filtered KG are all acyclic. In some embodiments, the filtered KG comprises an acyclic path that is a representation of a cyclic path or a portion thereof. In some embodiments, the filtered KG does not comprise a cyclic path. In some embodiments, paths connecting a stimulus and a condition are limited based on length. In some embodiments, the path length, as defined by the number of causal edges, of paths of a stimulus-condition KG is 10 or fewer causal edges, such as any of 9 or fewer causal edges, 8 or fewer causal edges, 7 or fewer causal edges, 6 or fewer causal edges, 5 or fewer causal edges, 4 or fewer causal edges, 3 or fewer causal edges, or 2 or fewer causal edges. In certain aspects, the application of a maximum path length in a KG database format allows for information contained in a KG, such as stimulus nodes, biological entity nodes, condition nodes, and causal edges to be more quickly and efficiently accessed and analyzed on the device, such as the computer.

In some embodiments, the method comprises obtaining (such as generating) a filtered KG. In some embodiments, the filtered KG is obtained from filtering a stimulus-condition KG. In some embodiments, the filtered KG is obtained from filtering another filtered KG. In some embodiments, the method comprises storing a filtered KG. In some embodiments, the method comprises displaying a visual representation of a filtered KG. In some embodiments, the visual representation of the filtered KG is interactive, *e*.*g*., a user may change views to view certain aspects of a filtered KG or subsections of the filtered KG. In some embodiments, the visual representation of the filtered KG is overlaid with another KG, such as a master KG, a stimulus-condition KG, or another filtered KG.

### B. Filtering

In certain aspects, the methods described herein comprise filtering a KG using signature data, such as stimulus signature data or condition signature data. Generally speaking, a filtering step applies a context-specific lens to a KG such that a biological pathway, and/or components thereof, connecting a stimulus and a condition can be identified with higher confidence and precision with regard to biological context, *e.g.,* as compared to text mining approaches, predictive approaches, and/or non-context-specific approaches.

As discussed herein, in certain aspects, the causal relationship represented by a causal edge is directional (*e.g.,* a first biological entity represented by a first biological entity node activates or inhibits a second biological entity represented by a second biological entity node). In some embodiments, the directionality of a causal edge is in the direction of a stimulus to a condition. Thus, in some embodiments, the causal edge implies a result exhibited by a biological entity or a condition represented by a node based on the causal relationship leading to that node. For example, in terms of expression (such as gene expression, including RNA expression and/or polypeptide expression), in some embodiments, the stimulus represented by a stimulus node upregulates expression of a biological entity represented by a biological entity node; in such an instance, the causal edge between the stimulus node and the biological entity node would reflect the stimulus activating the biological entity. In some embodiments, the stimulus represented by a stimulus node downregulates expression of a biological entity represented by a biological entity node; in such an instance, the causal edge between the stimulus node and the biological entity node would reflect the stimulus inhibiting the biological entity. In some embodiments, the first biological entity represented by a first biological entity node upregulates expression of a second biological entity represented by a second biological entity node; in such an instance, the causal edge between the first biological entity node and the second biological entity node would reflect the first biological stimulus activating the second biological entity. In some embodiments, the first biological entity represented by a first biological entity node downregulates expression of a second biological entity represented by a second biological entity node; in such an instance, the causal edge between the first biological entity node and the second biological entity node would reflect the first biological stimulus inhibiting the second biological entity. Based on the teachings provided herein, one will readily appreciate that this logic is readily extrapolated to the various stimuli, biological entities, and conditions that can be represented by a node described herein. For example, in terms of a post-translation modification of a polypeptide, in some embodiments, the stimulus represented by a stimulus node increases the presence of a biological entity having the post-translation modification represented by a biological entity node; in such an instance, the causal edge between the stimulus node and the biological entity node would reflect the stimulus activating the biological entity. In some embodiments, the stimulus represented by a stimulus node decreases the presence of a biological entity having the post-translation modification represented by a biological entity node; in such an instance, the causal edge between the stimulus node and the biological entity node would reflect the stimulus inhibiting the biological entity. In terms of a condition, such as a disease, in some embodiments, the biological entity represented by a biological entity node increases the presence or severity of some aspects of a condition, such as a symptom, represented by a condition node; in such an instance, the causal edge between the biological entity node and the condition node would reflect the biological entity activating the condition. In some embodiments, the biological entity represented by a biological entity node decreases the presence or severity of some aspects of a condition, such as a symptom, represented by a condition node; in such an instance, the causal edge between the biological entity node and the condition node would reflect the biological entity inhibits the condition.

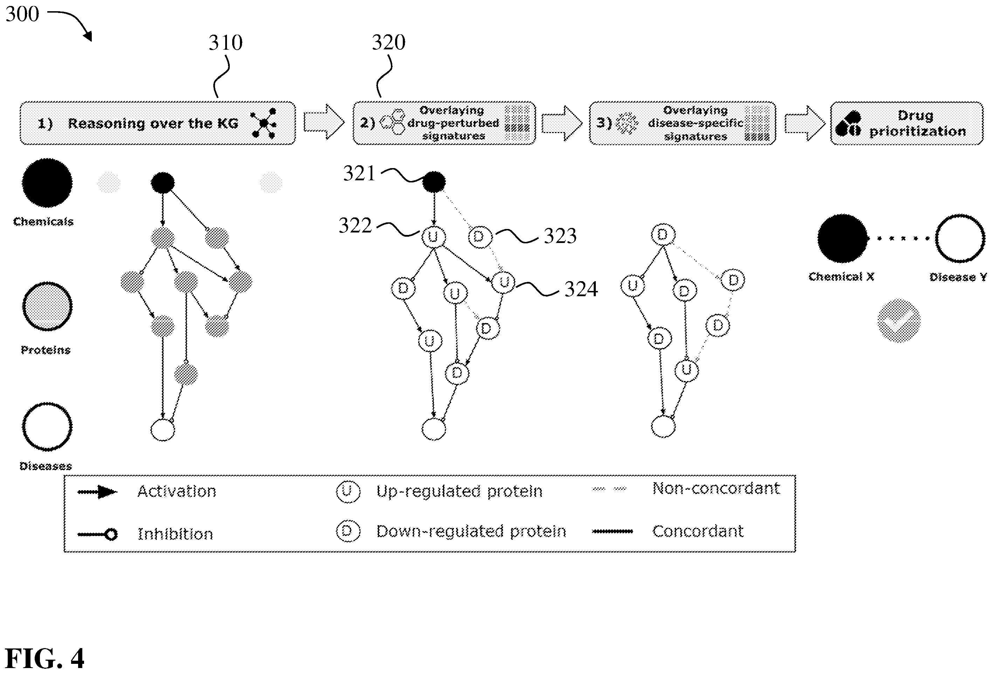

In some embodiments, the method comprises filtering a KG using signature data, wherein filtering is based on obtaining (*e.g.,* determining or identifying or selecting) one or more qualifying paths where the signature data is in agreement, at least to a degree, with an implied result of the stimulus node(s), biological entity node(s), and condition node(s) based on the causal edge leading thereto. As discussed herein, it is important to note that agreement is assessed in view of the signature data being applied to a KG for the filtering to identify qualifying path of the paths of the KG. For example, in some embodiments, the qualifying path is in agreement with drug signature data when each causal edge of the path is concordant with the drug signature data. In some embodiments, the qualifying path is in agreement with disease signature data when the disease signature data is opposite (anti-correlate) of the drug signature data and/or the implied results of the biological entity node(s) and/or disease node(s) based on causal edges directional from a drug node to a disease node. Thus, the filtered KGs described herein comprise one or more qualifying paths that are not removed during the filtering process. In some embodiments, paths removed during the filtering process (and thus no longer included in downstream filtered KGs) are not in agreement, at least to a degree, with signature data. For illustrative purposes, the concept of filtering is further described using an illustrative example. As shown in the schematic **300** of **FIG. 4****,** a stimulus-condition (drug-disease) KG is obtained by reasoning over a master KG **310** based on input of a drug and disease of interest. Subsequently, drug signature data from drug-perturbed information is used to filter the drug-disease KG **320.** Specifically, the drug signature data is overlaid with the drug-disease KG such that paths can be obtained having agreement between the drug-disease KG and the drug signature data. For example, as shown in **FIG. 4****,** the drug node **321** in the KG is connected to a first biological entity node **322** with a causal edge indicating that the drug represented by the drug node **321** activates the first biological entity represented by the first biological entity node **322.** The drug signature data shows that the first biological entity represented by the first biological entity node **322** is upregulated in the drug-context specific perturbation data, and thus the causal edge between the drug node **321** and the first biological entity node **322** is concordant. As shown in **FIG. 4****,** the drug node **321** in the KG is connected to a second biological entity node **323** with a causal edge indicating that the drug represented by the drug node **321** inhibits the second biological entity represented by the second biological entity node **323.** The second biological entity node **323** is connected to a third biological entity node **324** with a causal edge indicating that the second biological entity represented by the second biological entity node **323** activates the third biological entity represented by the third biological entity node **324.** The drug signature data shows that the second biological entity represented by the second biological entity node **323** is downregulated, and the third biological entity represented by the third biological entity node **324** is upregulated. While downregulation of the second biological entity of the second biological entity node **323** in the presence of the drug was implied by the KG and to be expected, upregulation of the third biological entity of the third biological entity node **324** was not implied by the KG when the second biological entity is downregulated. Thus, the causal edge between the drug node **321** and the second biological entity node **323** is concordant, and the causal edge between the second biological entity node **323** and the third biological entity node **324** is non-concordant. The path between the drug node and the disease node via the second biological entity node **323** is not in full agreement with the drug signature data, and in this instance the path is removed and not further analyzed, such as via filtering based on disease-specific data.

In some embodiments, it is possible to describe concordance and non-concordance in alternate terms based on a result that occurs to the entity represented by a node and not via description based on the causal edge. Such techniques are also encompassed by the description provided herein.

In some embodiments, filtering comprises obtaining (*e.g*., determining or identifying or selecting) a qualifying path where the signature data is in agreement, at least to a degree, with an implied result associated with the biological entity node(s) and condition node(s) based on the causal edge leading thereto. In some embodiments, the qualifying path is in full agreement with the signature data (*i.e.,* the implied result associated with the biological entity node(s) and condition node(s) matches the signature data for each node). In some embodiments, the filtering approach allows for one or more errors, such that a qualifying path is not remove from further analysis due to the presence of the one or more errors. For example, in some embodiments, the signature data overlaid on a KG results in a path having one or more non-concordant causal edge, wherein such path is not removed from further analysis and is including as a qualifying path in a filtered KG. In some embodiments, the filtering approach allows for 5 or fewer errors, such as any of 4 or fewer errors, 3 or fewer errors, 2 or fewer errors, or 1 error, before removing a path from further analysis. In some embodiments, the filtering approach allows for 1 error before removing a path from further analysis. In some embodiments, error is based on a percentage of non-concordant causal edges in a path, such that a qualifying path has 40% or less, such as any of 35% or less, 30% or less, 25% or less, 20% or less, 15% or less, or 10% or less, error. In some embodiments, the error is based on counter opposing information or a lack of information in signature data.

In certain aspects, removal of a path from further analysis does eliminate all nodes and/or causal edges of the path from the biological pathway connecting a stimulus and a condition. A biological pathway connecting a stimulus and a condition may comprise a plurality of paths, wherein two or more paths comprise overlapping nodes and/ or causal edges. In some embodiments, the filtering process only removes nodes and /or causal edges from a KG that can no longer be a part of any path of a biological pathway due to the presence of one or more non-concordant causal edges. For example, as illustrated in **FIG. 4** and described herein, the path encompassing the drug node **321** and biological entity nodes **323** and **324** comprises a non-concordant causal edge between the second biological entity node **323** and the third biological entity node **324.** As the second biological entity node **323** is isolated from other nodes, it is filtered out and removed as it is not in full agreement with the drug perturbed signature data. The third biological entity node **324** remains in the filtered KG as it is a part of another path in full agreement with the drug perturbed signature data.

### C. Signature data

In certain aspects, the methods described herein comprise the use of signature data (in certain aspects, the signature data is also referred to herein as perturbation data). Generally speaking, signature data is context-specific data related to a stimulus of a stimulus node or a condition of a condition node. In some embodiments, the signature data comprises information regarding the amount and/or one or more functions of the biological entity. In some embodiments, the signature data comprises upregulation and/or downregulation information. In some embodiments, the signature data is based on transcriptomic data. In some embodiments, the signature data is based on proteomic data.

In some embodiments, the signature data is stimulus signature data. In some embodiments, the stimulus signature data reflects changes in biological entities and/ or conditions due to a stimulus. For example, the stimulus signature data may comprise transcriptomic data from a cell exposed to a stimulus as compared to a reference, such as the cell not exposed to the stimulus.

In some embodiments, the signature data is condition signature data. In some embodiments, the condition signature data reflects changes in biological entities due to a condition. For example, the condition signature data may comprise transcriptomic data from a cell exposed to the condition, such as having the disease, as compared to a reference, such as the cell not exposed to the condition.

In some embodiments, the signature data comprises binary information, *e*.*g*., whether a biological entity represented by a biological entity node is upregulated (*e.g*., increase in observed expression) or downregulated (*e.g*., decrease in observed expression). In some embodiments, the signature data is based on a cut-off threshold. For example, in assessing whether a biological entity represented by a biological entity node is upregulated or downregulated, the signature data may require the assignment of upregulation to be at least a 2-fold increase, and downregulation to be at least 2-fold decrease. In some embodiments, in assessing whether a biological entity represented by a biological entity node is upregulated or downregulated, the signature data may require the assignment of upregulation to be at least a 1 or 2 log2-fold increase, and downregulation to be at least a 1 or 2 log2-fold decrease.

In some embodiments, the signature data (such as stimulus signature data or condition signature data) comprises information obtained from text mining, a database, and/ or a proprietary source. In some embodiments, the database is one or more of CREEDS (*see,* Wang *et al.,* 2016), L100 (*see,* Subramanian *et al.* 2017), Open Targets (*see,* Ochoa *et al.,* 2021), and GEO *(see,* Barrett *et al.,* 2012). In some embodiments, the information is an experimental source, such as a transcriptomic or proteomic study. In some embodiments, the stimulus signature data comprises information obtained from a stimulus-perturbation experiment. In some embodiments, the condition signature data comprises information obtained from a condition-perturbation experiment.

### D. Stimuli and conditions

The method, and aspect thereof, described herein can be used to study a diverse array of stimuli and conditions. In some embodiments, the stimulus is a medical intervention, such as a drug or treatment. In some embodiments, the stimulus is a drug. In some embodiments, the stimulus is a plant extract. In some embodiments, the stimulus is an environmental stimulus, such as a toxin or pollutant. In some embodiments, the stimulus is based on a personal-choice or exposure, such as a smoking. In some embodiments, the stimulus is an infection, such as a bacterial or viral infection. In some embodiments, the stimulus is a genetic alteration, such as a gene mutation. In some embodiments, the condition is a disease or disorder. In some embodiments, the condition is a phenotype. In some embodiments, the condition is a side effect. In some embodiments, the condition is a symptom, such as a symptom associated with a disease.

In certain aspects, it may be helpful to characterize a stimulus and a condition as a pair, *e.g.,* a drug-disease pair or a plant extract-disease pair. In some embodiments, the stimulus-condition pair are known to be associated, but the biological pathway, or an aspect thereof, are not fully characterized or known. In some embodiments, the stimulus-condition pair are not known to be associate, wherein the methods described here may be useful for identifying a biological pathway connecting the stimulus and the condition.

In some embodiments, the stimulus and/or condition are associated with an individual. For example, in some embodiments, the stimulus is a drug, and the drug is to be administered to an individual. In some embodiments, the individual is a human.

### E. Additional aspects of the methods

In certain aspects, the methods described herein further comprises steps performed upstream and/or downstream of one or more filtering steps.

In some embodiments, the method comprises scoring paths of an identified biological pathway, such as a biological pathway connecting a stimulus and a condition. In such embodiments, the biological pathway comprises a plurality of paths connecting the stimulus and the condition. In some embodiments, the plurality of paths may be scored based on a feature of a path such as to predict the strength of the connection between the stimulus and the condition via the path, *e.g.,* the number of causal edges of the path, the number of repeating nodes, the number of causal edge errors in the path based on the signature data, the type of causal edge error, and the degree to which one or more nodes of the path are upregulated and/or downregulated according to the signature data. For example, in some embodiments, when comparing two paths connecting a stimulus and a condition, the path having fewer causal edges would be ranked higher than the path having more causal edges. In some embodiments, the plurality of paths may be scored based on similarity to a known biological pathway such as the Wnt-signaling pathway. Information regarding members of pathways, such as proteins in a known biological pathway is available in the art, such as from publicly available databases.

In some embodiments, the method comprises scoring a plurality of biological pathways against one another. For example, in some embodiments, the methods described herein are used to identify a biological pathway for each of a group of drug-disease pairs, wherein the drug of each pair is different and the disease of each pair is the same (*e.g.*, assessing a number of drugs for treating the same disease). In some embodiments, the method comprises scoring a plurality of biological pathways based on the associated path(s) constituting each biological pathway. In some embodiments, the plurality of biological pathways is scored (or ranked) based on the number of paths of the biological pathway. For example, a first biological pathway comprising 2 paths from a first drug to the disease would be ranked higher than a second biological pathway comprising 1 path from a second drug to the disease. In some embodiments, the plurality of biological pathways are scored based on the quality of the path(s) constituting each biological pathway. In some embodiments, the quality of a path is assessed via the number of causal edges of the path, the number of repeating nodes, the number of causal edge errors in the path based on the signature data, the type of causal edge error, and the degree to which one or more nodes of the path are upregulated and/or downregulated according to the signature data. In some embodiments, the plurality of biological pathways may be scored based on similarity to a known biological pathway such as the Wnt-signaling pathway. Information regarding members of pathways, such as proteins in a known biological pathway is available in the art, such as from publicly available databases.

In some embodiments, the method comprises identifying a target biological entity based on an identified biological pathway for further assessment. In some embodiments, the method comprises scoring biological entities constituting a biological path. In the context of drug discovery, in some embodiments, the biological entity, such as a protein target, may be scored and identified based on a characteristic of the biological pathway (*e.g.,* the number of repeats of the biological entity in the biological pathway or an indication from the signature data, *e.g.,* a strongly upregulated component) or other information such as drugability of the target.

In some embodiments, the method comprises validating a biological pathway, or an aspect thereof, via an experimental technique or study, such as a biochemical, pharmaceutical, or clinical technique or study.

### F. Applications of the methods, and aspects thereof, described herein

The methods, and aspects thereof, described herein have a number of different applications and uses.

In some embodiments, provided is a method of identifying a biological pathway that connects a stimulus and a condition (such as a drug and a disease).

In some embodiments, provided is a method of identifying a drug that is useful for treating and/or preventing a condition, such as a disease. In such embodiments, a plurality of drugs may be assessed to see if a biological pathway exists between a drug and the condition.

In some embodiments, provided is a method of identifying a stimulus that is associated, such as responsible to some degree, with a condition, such as a disease. In such embodiments, a plurality of stimuli may be assessed to see if a biological pathway exists between a stimulus and the condition.

In some embodiments, provided is a method of drug prioritization comprising assessing a plurality of drugs for use in treating a condition using the methods described herein, and ranking the results to identify one or more drugs for further study. In some embodiments, ranking comprises comparing a first biological pathway of a first drug and the condition with a second biological pathway of a second drug and the condition.

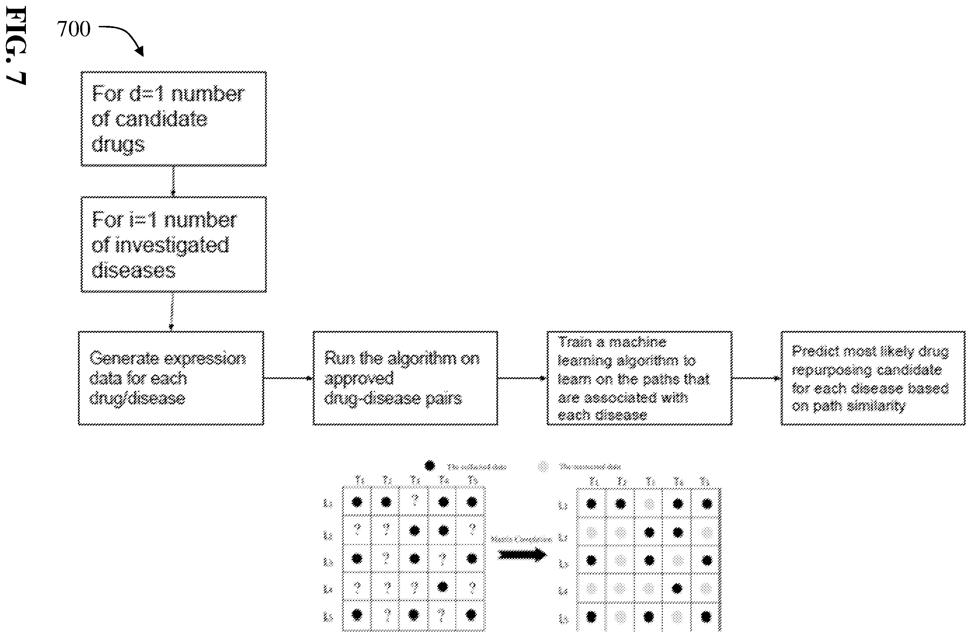

In some embodiments, provided herein is a method for drug repurposing, such as identifying new disease or patient population (including patient subpopulations) for a known drug can provide a therapeutic effect. In such embodiments, the drug may be assessed to see if a biological pathway exists between the drug and one or more of a disease and/or patient population. A schematic of a workflow for drug repurposing using the method described herein is provided in **FIG. 7****.**

In some embodiments, provided is a method of identifying a target biological entity for further studies, such as SAR optimization or drugability. In such embodiments, the method comprises identifying a biological pathway that connects a stimulus and a condition (such as a drug and a disease), and evaluating (such as scoring) the biological entities of the biological pathway to identify the target biological entity.

In some embodiments, provided is a method of conducting a time-based analysis of the interaction between a stimulus and a condition. For example, the methods enable the study of changes of a biological pathway between a drug and a disease over time, such as by filtering using signature data acquired at different time points.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced within the scope of the present disclosure.

### III. Exemplary methods

In certain aspects, provided herein is a method of identifying a biological pathway that connects a drug and a disease in an individual, the method performed at a system comprising one or more processors, the method comprising: filtering a drug-disease knowledge graph using drug signature data to obtain a drug-filtered knowledge graph, wherein the drug-disease knowledge graph comprises: (a) nodes, each node representing the drug, biological entity, or the disease; and (b) causal edges, each causal edge representing a causal relationship connecting two nodes, wherein the drug signature data comprises information pertaining to biological entities associated with the drug, and wherein the drug-filtered knowledge graph comprises one or more qualifying paths according to the drug signature data; filtering the drug-filtered knowledge graph using disease signature data to obtain a disease-filtered knowledge graph to identify the biological pathway that connects the drug and the disease, wherein the disease signature data comprises information pertaining to biological entities associated with the disease, and wherein the disease-filtered knowledge graph comprises one or more qualifying paths according to the disease signature data. In some embodiments, the identified biological pathway that connects the drug and the disease is indicative that the drug is useful for treating the disease.

In other aspects, provided here in a method of identifying a biological pathway that connects a drug and a disease in an individual, the method performed at a system comprising one or more processors, the method comprising: filtering a drug-disease knowledge graph using disease signature data to obtain a disease-filtered knowledge graph, wherein the drug-disease knowledge graph comprises: (a) nodes, each node representing the drug, biological entities, or the disease; and (b) causal edges, each causal edge representing a causal relationship connecting two nodes, wherein the disease signature data comprises information pertaining to biological entities associated with the disease, and wherein the disease-filtered knowledge graph comprises one or more qualifying paths according to the disease signature data; and filtering the disease-filtered knowledge graph using drug signature data to obtain a drug-filtered knowledge graph to identify the biological pathway that connects the drug and the disease, wherein the drug signature data comprises information pertaining to biological entities associated with the drug, and wherein the drug-filtered knowledge graph comprises one or more qualifying paths according to the drug signature data. In some embodiments, the identified biological pathway that connects the drug and the disease is indicative that the drug is useful for treating the disease.

In other aspects, provided herein is a method of identifying a biological pathway that connects a plant extract (or a component thereof) and a disease in an individual, the method performed at a system comprising one or more processors, the method comprising: filtering a plant extract-disease knowledge graph using plant extract (or a component thereof) signature data to obtain a plant extract-filtered knowledge graph, wherein the plant extract-disease knowledge graph comprises: (a) nodes, each node representing the plant extract (or a component thereof), biological entity, or the disease; and (b) causal edges, each causal edge representing a causal relationship connecting two nodes, wherein the plant extract signature data comprises information pertaining to biological entities associated with the plant extract (or a component thereof), and wherein the plant extract-filtered knowledge graph comprises one or more qualifying paths according to the plant extract signature data; filtering the plant extract-filtered knowledge graph using disease signature data to obtain a disease-filtered knowledge graph to identify the biological pathway that connects the plant extract (or a component thereof) and the disease, wherein the disease signature data comprises information pertaining to biological entities associated with the disease, and wherein the disease-filtered knowledge graph comprises one or more qualifying paths according to the disease signature data. In some embodiments, the identified biological pathway that connects the plant extract (or a component thereof) and the disease is indicative that the plant extract (or the component thereof) is useful for treating the disease.

In other aspects, provided herein is a method of identifying a biological pathway that connects a plant extract (or a component thereof) and a disease in an individual, the method performed at a system comprising one or more processors, the method comprising: filtering a plant extract-disease knowledge graph using disease signature data to obtain a disease-filtered knowledge graph, wherein the plant extract-disease knowledge graph comprises: (a) nodes, each node representing the plant extract (or a component thereof), biological entities, or the disease; and (b) causal edges, each causal edge representing a causal relationship connecting two nodes, wherein the disease signature data comprises information pertaining to biological entities associated with the disease, and wherein the disease-filtered knowledge graph comprises one or more qualifying paths according to the disease signature data; and filtering the disease-filtered knowledge graph using plant extract signature data to obtain a plant extract-filtered knowledge graph to identify the biological pathway that connects the plant extract (or a component thereof) and the disease, wherein the plant extract signature data comprises information pertaining to biological entities associated with the plant extract (or a component thereof), and wherein the plant extract-filtered knowledge graph comprises one or more qualifying paths according to the plant extract signature data. In some embodiments, the identified biological pathway that connects the plant extract (or a component thereof) and the disease is indicative that the plant extract (or the component thereof) is useful for treating the disease.

### EXAMPLES

The following examples are included for illustrative purposes and are not intended to limit the scope of the teachings provided in the present application.

### Example 1

This example demonstrates methodology described herein for prioritizing drugs for a particular disease by reasoning over causal paths in a knowledge graph (KG), guided by both drug-perturbed and disease-specific transcriptomic signatures. As demonstrated herein, this methodology was able to recover a large proportion of clinically investigated drug-disease pairs on multiple transcriptomic datasets and KGs, and performed better than other known network-based methods. Furthermore, two additional applications were demonstrated to illustrate how this approach can also assist in hypothesis generation and target prioritization.

Network-based approaches are becoming increasingly popular for drug discovery as they provide a systems-level overview of the mechanisms underlying disease pathophysiology. They have demonstrated significant early promise over other methods of biological data representation, such as in target discovery, side effect prediction, and drug repurposing. However, existing approaches have significant limitations and drawbacks. For example, existing approaches often cannot be directly used for target discovery, side effect prediction, and drug repurposing, and are an indirect analysis tool. Furthermore, existing approaches are designed for bipartite graphs, thus, requiring simplification of biological pathways to a single relation between two proteins which leads limited utility. Analysis of large large-scale networks, such as KGs, is complex and often time intensive as KGs can grow exponentially to the point where it is highly likely that a KG contains information that may not be relevant in a true biological context. This is especially of concern with the current explosion of *-omics* data from the deep characterization of biological systems. The approaches described herein provide solutions to setbacks encountered with existing methodology, and provide a manner to ensure a proper level of biological context.

The representation of bimolecular interactions occurring within cells is often intuitively organized in the form of biological networks. These networks can be used to inherently model biological processes via the development of knowledge graphs (KGs), which use of nodes denoting, *e*.*g*., biological entities and edges representing their relationships. While homogeneous networks, such as protein-protein interaction networks, represent relationships between a single entity type, knowledge graphs (KGs) as described herein may incorporate a broad range of biological scales, from the genetic and molecular level (e.g., proteins, drugs, and biochemicals), to biological concepts (e.g., phenotypes and diseases). The KGs described herein were then utilized for several applications in drug discovery, such as providing insights into molecular mechanisms and therapeutic targets, side effect prediction in the early stages of drug development, target prioritization, and drug repositioning.

Given the flexibility of KGs, multiple heterogeneous relation types were modeled to represent biological processes that are governed by interactions occurring between component entities. Even though a variety of relation types (e.g., literature co-occurrence, associations) can be leveraged by network-topology algorithms for various applications, causal relations were identified as particularly useful as they can be used to infer the effect of any given node on another by reasoning over the KG. Nonetheless, not all interactions included in a given KG are necessarily biologically relevant as they may be context-specific, such as to a particular cell type, tissue or disease. Furthermore, as the complete human interactome remains unknown, KGs modeling protein-protein interactions (PPIs) are also incomplete and the interactions which are modeled tend to be biased towards well studied proteins and their relationships. The methodology described herein integrated mapping the signatures of an *-omics* experiment to a KG, we can not only verify which causal interactions are observed within a specific context, but also prioritize and identify the mechanism of action of a drug for a given disease with high precision.

In brief, the approach described herein identified the causal paths that connect a drug to a particular disease. Next, reasoning (also referred to herein as filtering) was performed over these paths to identify those that correlate with the transcriptional signatures observed in a drug-perturbation experiment, and anti-correlate to signatures observed in the disease of interest. The paths that match the signature data are then proposed to represent the mechanism of action of the drug in the context of the disease. The methodology described herein consistently prioritized clinically investigated drug-disease pairs on multiple datasets and KGs, achieving better performance over other similar methodologies.

### Results

This section is divided into three subsections that outline the different applications of the methodology presented herein. In subsection 1, we demonstrated how the methodology can be used to identify potential drug candidates for various diseases using a variety of KGs and datasets, outperforming numerous link prediction methods. In subsection 2, the inherent interpretability of KGs was leveraged to generate hypotheses for the predictions made by the methodology. In subsection 3 it was demonstrated how the methodology can be reversed-engineered and alternatively used to predict targets for a given disease.

### 1. Identification of drug candidates

To demonstrate the ability of the methodology to accurately identify drug candidate for a given disease, the methodology was evaluated for its performance to recover clinically investigated drug-disease pairs using two distinct KGs and four transcriptomic datasets (i.e., two each containing numerous drug-perturbed and disease transcriptomic experiments). In this task, the methodology consistently prioritized a significantly larger number of clinically investigated drug-disease pairs across all datasets and in both KGs compared with the precision expected by chance (i.e., probability of randomly picking a true positive among all possible drug-disease combinations) **(Table 1).** In **Table 1,** each row corresponds to the results of running the methodology on a specific drug-disease dataset combination, and the second and fourth columns show the performance that is expected to be achieved by chance. The highest precision values were found for the L1000-GEO datasets with 80% and 66.67% for the OpenBioLink and custom KGs, respectively. In the remaining datasets, the precision was approximately 50%, except for the CREEDS-Open Target datasets in the custom KG that exclusively yielded a single drug-disease pair which was not in clinical trials. While the precision expected by chance approximately varied between 10% and 35%, the methodology consistently achieved higher precision values across nearly all datasets and KGs, ranging between 50% and 80% (e.g., more than five times higher for the L1000-GEO dataset in OpenBioLink running RPath (80%) vs. chance (15.66%)). Notably, the number of prioritized drug-disease pairs were constrained for two reasons: i) the methodology required transcriptomic information for a given drug and disease and, ii) the pair must also be present in the KG. Furthermore, apart from the low number of drug-disease pairs that fulfilled these criteria, the methodology filtered the majority of the drug-disease pairs with paths between them after overlaying the transcriptomic signatures in Steps 1 and 2 **(****FIG. 4****).** For example, in the case of the CREEDS-GEO datasets and the OpenBioLink KG, the total number of diseases was 10, resulting in only a couple of drug-disease pairs being prioritized. Nonetheless, it was still possible to validate the methodology across multiple datasets and KGs, observing that the methodology performed significantly better than chance at identifying clinically investigated drug-disease pairs.

**Table 1. Evaluation of the methodology across two KGs using precision.**

| | **OpenBioLink KG** | | **Custom KG** | |
|---|---|---|---|---|
| **Dataset combination** | **Precesion (TP/TP+FP)** | **Expected precision by chance** | **Precesion (TP/TP+FP)** | **Expected precision by chance** |
| L1000-GEO | 80% (4/5) | 15.66% | 66.67% (2/3) | 12.83% |
| L1000-Open Target | 54.55% (6/11) | 11.32% | 50% (2/4) | 9.23% |
| CREEDS-Open Target | 50% (1/2) | 26.17% | 0% (0/1) | 24.36% |
| CREDS-GEO | 50% (1/2) | 36.77% | 50% (1/2) | 33.52% |

Additionally, the methodology was benchmarked against 11 alternative methods that have been used to predict drug-disease links in a KG with the same characteristics as the ones used in this work (Abbas *et al.,* 2021; Zietz *et al.,* 2020). The precision of these methods varied between 5% and 43%. Furthermore, since the majority of these methods prioritize a drug and a disease based on their network proximity (e.g., shortest paths and number of shared nodes), these methods recurrently prioritized the same set of drug-disease pairs. Thus, these methods could not be used to prioritize drugs outside the vicinity of disease-associated proteins since only a minority of drug-disease pairs are connected by a single protein, but the majority of them contain longer paths that are not considered by these methods.

### 2. Interpretation of the mechanisms of action of the proposed drug candidates

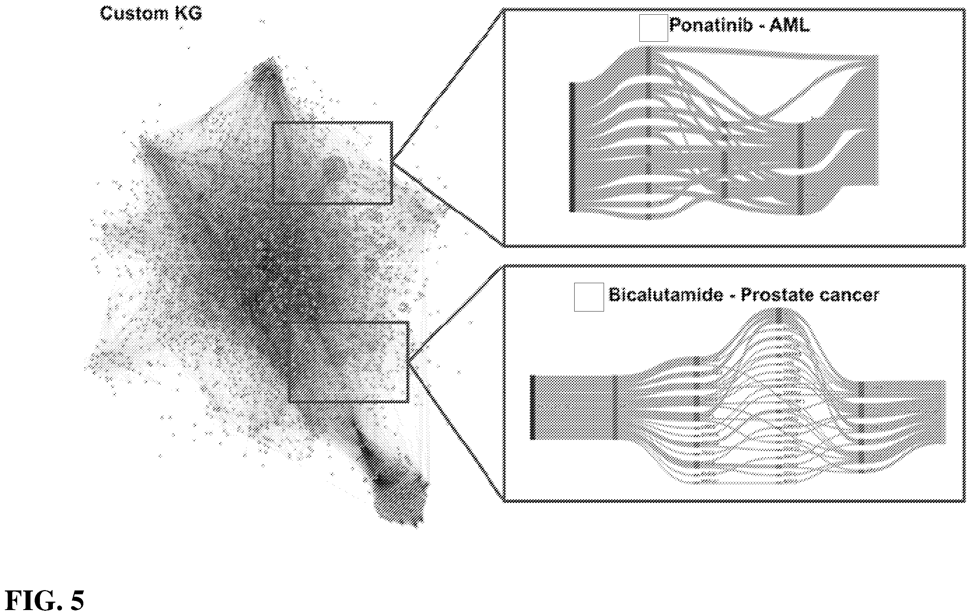

The paths between two of the validated drug-disease pairs that were prioritized by the methodology described herein were explored to demonstrate how this approach can be used to deconvolute the mechanism of action of a given drug **(****FIG. 5****).** Bicalutamide and ponatinib, two anti-cancer drugs already approved for prostate cancer and acute myeloid leukemia, respectively, were selected for further study as the mechanisms of action of these drugs have been widely studied and allow for the comparison of the mechanistic paths identified by the methodology against known interactions and pathways reported in scientific literature.

Ponatinib, a multi-targeted tyrosine-kinase inhibitor, is used to treat acute myeloid leukaemia (AML). Among the targets of this drug present in the qualifying paths for this pair, the methodology was able to identify fms-like tyrosine kinase 3 (FLT3), which is mutated in approximately 20% of AML patients (Smith *et al.,* 2012) and several members of the FGFR family proteins. Furthermore, other proteins including KDR, LYN, and SRC, all of which are kinase-associated targets in AML were observed. As a downstream target of these proteins, JAK2, a well-studied player in myeloproliferative diseases, with known mutations and hypermethylation events was also observed. The transcription factor, CEBPA, that is critical for normal development of granulocytes and is also implicated in AML (Pabst and Mueller, 2009) and the SPI1 gene, from which circSPI1, a circular RNA derived from the gene, has recently been shown to be highly expressed in AML patients (Wang *et al.,* 2021) were also identified. Other proteins that are inhibited as a result of the signaling cascade triggered by ponatinib include KIT, which is implicated in cell death in AML (Heo *et al.,* 2017). RAS family members NRAS and KRAS, both of which are associated with the prognosis of solid tumors and hematological malignancies, including AML (Mascaux *et al.,* 2005) were also implicated.

The second studied drug-disease pair is bicalutamide (*brand name* CasodexTM), used for the treatment of prostate cancer. Bicalutamide is an anti-androgen medication that binds to the androgen receptor (AR). The qualifying paths between bicalutamide and prostate cancer point to several downstream targets of this drug, including the epigenetic regulator KMT2D, which is known to sustain prostate carcinogenesis by epigenetic mechanisms (Ly *et al.,* 2009), and NECAB3, known to enhance the activity of HIF1A, thus promoting glycolysis under normoxic conditions and enhancing tumorigenicity in cancer cells (Nakaoka *et al.,* 2016). Furthermore, CTNNB 1, which plays a role in the development of numerous prostate cancers (Gerstein *et al.,* 2002) was identified. Interestingly, novel players that have not yet been reported in the literature, such as GNAI1, SYMPK, UBR5, and MEF2C were also observed and may provide new insights on the mechanism of action of this drug.

### 3. Target prioritization

Prior to the identification of a therapeutic drug candidate for any given disease, a crucial first step is to often identify biologically relevant protein targets. Ideally, the perturbation of a particular protein target in a disease state should result in the reversal of the observed phenotype. In a similar manner to the above-mentioned applications, by reasoning over the KG guided by disease signatures, the methodology described herein was used for target prioritization. There are no large datasets that contain information about known targets for a wide variety of indications, and thus it was not possible to conduct a validation strategy similar to the analyses presented herein. Rather, for this study the results were evaluated based on the top prioritized protein targets across all diseases using literature evidence **(Table 2).** Among the top protein target-disease pairs proposed by the methodology, two have already been associated with AML, including PRKCA, for which several drugs already exist (Konopatskaya and Poole, 2010; Takami, M *et al.,* 2018), and CXCL8/IL-8 (Campbell *et al.,* 2013; Schinke *et al.,* 2015; Kuett *et al.,* 2015). Furthermore, CDC42, which has been proposed as a candidate target for medulloblastoma, plays a role in several cancers. Specifically, CDC42 has been shown to act as a regulator of medulloblastoma-associated genes (Nalla *et al.,* 2010) and compounds for its inhibition have also been proposed (Hong *et al.,* 2013).

**Table 2. Prioritized protein target-disease pairs.**

| **Protein target** | **Disease** | **Qualifying paths** | **Nodes in the qualifying paths** | **KG** | **Transcriptom ic dataset** |
|---|---|---|---|---|---|
| NOG | AML | 18,456 | 1,008 | Custom KG | GEO |
| PRKCA | AML | 12,861 | 669 | Custom KG | GEO |
| CXCL8/ IL-8 | AML | 7,234 | 465 | Custom KG | GEO |
| NOG | Plasma cell myeloma | 5,743 | 616 | Custom KG | GEO |
| CDC42 | Medulloblasto ma | 5,651 | 91 | OpenBioLink | GEO |

### Discussion

The demonstrations herein present a methodology that leveraged prior knowledge from causal relations across multiple biological modalities in KGs and assessed their correlation with transcriptomic signatures for drug discovery. As discussed above, known analytical techniques present shortcomings, including that: i) they operate on homogeneous causal graphs with a single entity type (e.g., protein nodes), ii) they are solely conducted on single contrast experiments (e.g., drug-treated vs. control), and iii) they do not fully exploit all possible paths in these causal graphs. The methodology demonstrated herein addressed these shortcomings by reasoning over all possible causal paths in a multimodal KG and leveraging both drug and disease transcriptomic signatures. Specifically, the methodology reasoned over the ensemble of paths between a given drug and a disease in a KG. Furthermore, the methodology evaluated the concordance of these paths against the transcriptomic changes experimentally observed for that drug. Additionally, the methodology assessed whether the effect of these paths is opposite to the transcriptomic signatures observed within the disease context. Such methodology enabled the identification of potential drug candidates as those whose paths correlate with drug-perturbed transcriptomic signatures and are anti-correlated to the disease transcriptomic signatures. As discussed above, the methodology was validated in eight independent analyses, finding that the methodology demonstrated herein consistently identifies a large proportion of clinically investigated drug-disease pairs over multiple datasets and KGs. Additionally, several robustness experiments were conducted and benchmarking the methodology against 11 network-based methodologies showed improved results. Moreover, the methodology demonstrated the capability of deconvoluting the mechanism of action of a drug as well as to prioritize protein targets for a given disease.

### Methodology

The methods described herein were used, in some aspects, to identify and prioritize drug-disease pairs. In brief, as illustrated in **FIG. 4****,** reasoning was performed in step 1 such that all paths of a given length between a drug node and a disease node in a master KG were calculated to generate a drug-disease KG. We denoted a KG as a set of nodes and edges, where nodes correspond to three distinct biological entities (i.e., chemicals, proteins, and diseases) connected through causal relations, representing activatory or inhibitory effects. Causal relations within the KG connect drug-protein, protein-protein, and protein-disease nodes. A (directed) path in a KG is a sequence of two or more biological entities connected through causal relations. Paths in the KG can be either cyclic or simple. A cyclic path refers to paths in which one or more nodes repeat, whereas a simple path corresponds to a path in which no nodes appear more than once. The length of a path is defined by the number of edges that connect the nodes within the path. Each of these paths can be divided into three main sequential parts that represent the mechanism of action of a drug: i) the drug activates/inhibits a protein target (drug-protein edge), ii) the protein target triggers a signaling cascade (a set of protein-protein edges), and iii) the signaling cascade reverts the disease condition (protein-disease edge). Furthermore, since every causal edge contains information on the effect each node exerts on another (activation or inhibition), we inferred the direction of regulation (up-/down-regulated) for each node at each step of a path. If there existed a causal acyclic path connecting the drug and the disease, a subgraph involving all these paths was inferred. This subgraph represents the proposed mechanism of action by which the drug might be a therapeutic target of the given disease. In step 2, using the drug-disease KG, transcriptomic signatures observed from a drug-perturbed experiment were overlaid onto each corresponding node present in the paths of the drug-disease KG. The filtering process traversed through each path and evaluated whether the inferred direction of regulation (*i.e.,* activation or inhibition) at every step was concordant with the observed up-and down-regulations observed in the drug transcriptomic signature. Non-qualifying paths were removed to generate a drug-filtered KG, which was used in further steps of the method. In step 3, transcriptomic signatures observed within a specific disease context were overlaid onto each node in the drug-filtered KG obtained from the previous step. In a similar manner as step 2, the filtering process traversed through each path and evaluated whether the inferred direction of regulation (*i.e.,* activation or inhibition) at every step was concordant with the observed up-and down-regulations of the disease signature data (*i.e.,* whether the disease transcriptomic signatures contradict the paths that were concordant with the drug signatures). Resulting paths were used to identifying drug-disease pairs and enable drug prioritization.

It was hypothesized that because a number of paths might represent the biologically relevant mechanism of action of this drug, the observed transcriptomic signatures for proteins in the KG should be concordant with the inferred up- or down-regulations at every step of the path. For example, if in a given path, a drug inhibits a protein target and that target activates a signaling cascade, we expected the inhibition of the protein target as well as the inhibition of the proteins downstream of the target. In this example, a gene (or associated expression product) was considered to be differentially expressed if its expression was significantly altered with respect to a reference sample (*i.e.,* control). To achieve this, a cut-off was applied to each measured gene (or associated expression product) in the experimental dataset based on the fold change such that this measurement was used to define whether the gene was up-/down-regulated or unchanged.

Furthermore, it was hypothesized that, in contrast to the overlaying of drug-perturbed signatures, transcriptomic signatures in a disease context should be anti-correlated to both the drug-perturbed signatures as well as the inferred up- or down-regulations for every node in the path. In summary, this methodology aimed at prioritizing a specific drug for a given disease if i) there existed causal paths between the drug and disease in the drug-disease KG, ii) the causal effects on these paths were aligned with the transcriptomic changes observed in the drug-perturbed experiment, and iii) both the drug signatures and the paths are anti-correlated with the transcriptomic dysregulations observed in the disease state.

It is noted that the process was also modified using the above logic to begin from the disease of interest to calculate paths from the disease to all proteins of a given path length *(e.g.,* a path length of 6). Using this process, the methodology calculated the concordance between the paths for each potential protein target and the transcriptomic signatures of the given disease to assess whether there were proteins that could be key up-stream regulators of the observed phenotype. This approach significantly increased the running time of the algorithm as it required querying paths from a disease to several thousands proteins in the KG.

For the conducted analyses, four databases were utilized. Drug-perturbed transcriptomic data were obtained from CREEDS (Wang *et al.,* 2016) and L1000 (Subramanian *et al.,* 2017), and disease transcriptomic data were collected from Open Targets (Ochoa *et al.,* 2021) and GEO (Barrett *et al.,* 2012). All experimental datasets from these resources (downloaded on February 15, 2021) were from gene expression changes measured in humans. Chemicals and diseases from datasets obtained from these databases were then mapped to PubChem compound identifiers and the Mondo Disease Ontology (MONDO), respectively, for consistency with the entities of the knowledge graphs presented in the next section. Similarly, gene identifiers in all datasets were harmonized to ENTREZ. Of the four databases, datasets from L1000 contained a binarized value for the direction of dysregulation for every gene (*i.e.,* up-regulation and down-regulation), while for the remaining databases, fold changes were binarized for significantly dysregulated genes using a cut-off of at least a log2 fold change. A systematic search was conducted for databases that contained either a large number of drug-perturbed or disease-specific transcriptomic datasets. This search initially resulted in 27 candidate databases, however, certain candidate were not pursued as they either contained too few transcriptomic datasets or the drugs/diseases in these datasets were not in the KGs used to demonstrate our methodology.

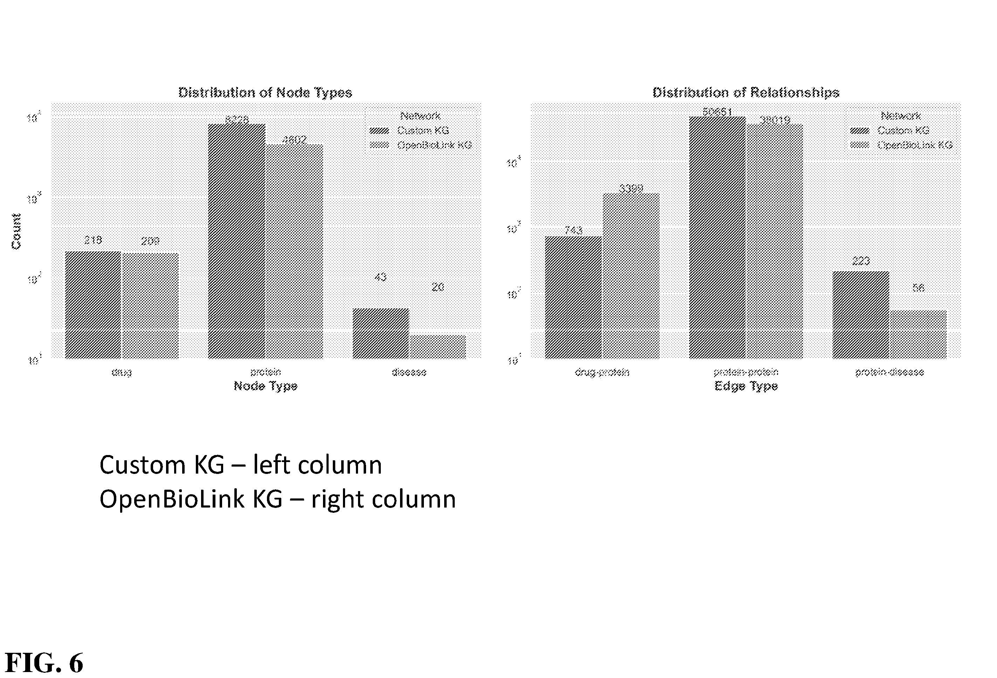

The OpenBioLink KG (Breit *et al.,* 2020) and a custom KG (Rivas-Barragan *et al.,* 2020) were both KGs originally generated from a compedia of independent databases; thus, containing unique causal interactions depending on the source databases they include. The KGs were reduced to encompass three types of causal edges: drug-protein (i.e., drug activates/inhibits protein), protein-protein (i.e., protein activates/inhibits protein), and protein-disease (i.e., protein activates/inhibits disease). Furthermore, the original node identifiers for chemicals and diseases in both KGs were respectively mapped to PubChem compound identifiers and MONDO to be consistent with the transcriptomic datasets. Next, drugs and diseases were removed that were not present in any of the four transcriptomic datasets presented in the previous subsection as the paths between these drug-disease pairs cannot be validated. **FIG. 6** shows the final statistics of both KGs after the previously outlined filtering steps.

When filtering based on the disease signature data, a qualifying path was allowed to have up to one error *(i.e.,* one error was allowed when calculating the anti-correlation in the path between a drug and a disease). Such allowance was made to limit the impact of an arbitrary fold change cut-off, which ultimately determines the up-/down-regulation of each protein, and as some paths might contain causal relations that do not reflect a change at the transcription level of the affected protein (e.g., phosphorylation of a protein kinase).

Aspects of the methodology were implemented in Python leveraging NetworkX (v2.5) (https://networkx.github.io). Network visualizations were done using WebGL, D3.js, Three.js, Matplotlib, and igraph.

As discussed herein, drug-disease pairs were used that have been clinically investigated as positive labels, extracting this information from ClinicalTrials.gov (accessed on September 28, 2020). Since drugs and diseases in ClinicalTrials.gov are formalized using MeSH identifiers, the identifiers were harmonized to the ontologies used in the KG (i.e., PubChem compound for chemicals and MONDO for diseases) using proprietary harmonization scripts. After the harmonization, any drug or disease in the KGs that was not present in any clinical trial or did not have any path to any disease in the KG was subsequently removed, as its corresponding node cannot be used in the presented validation.

To test the robustness of the methodology in identifying these clinically investigated drug-disease pairs, eight independent analyses were conducted, one for each of the combinations of the two drug datasets, the two disease datasets, and the two KGs (e.g., CREEDS-GEO-OpenBioLink, LINCS-GEO-OpenBioLink, etc.). For each of these eight analyses, the methodology was performed over a given KG to identify prioritized drug-disease pairs, in other words, pairs whose paths are both correlated with drug-perturbed transcriptomic signatures and anti-correlated with disease transcriptomic signatures. Two assumptions in the design of the methodology were made for this demonstration. Firstly, paths with cycles or a length greater than 7 edges between a given drug and disease were not considered, assuming that the effects exerted by paths beyond this length are less biologically relevant. Secondly, at most one error between the transcriptomic data and a given path was allowed for. In this set of prioritized drug-disease pairs, it was expected that a larger proportion of clinically investigated drug-disease pairs (i.e., positive labels) would be retriebed than expected by chance (i.e., proportion of positive labels in the dataset). Here, it is important to note that i) as in any drug discovery task, there is a class label imbalance (i.e., the vast majority of the drug-disease pairs are negative labels), and ii) since each dataset contains a different set of chemicals/diseases, each of the eight analyses has a different proportion of positive labels (between 1% and 15%). Furthermore, this type of validation falls into the so-called early retrieval problem. In other words, from the thousands of drug-disease pairs that were tested, the validatino exclusively prioritized the top-ranked pairs that have been equally prioritized by the methodology. This small subset represents the interesting drug-disease pairs that would be further investigated in the drug discovery process. In such cases, it was inadequate to apply metrics such as receiver operating characteristic (ROC) curves (i.e.. AUC-ROC and AUC-PR) as they operate on a full ranked list. Therefore, it does not necessarily evaluate the ability of a model to prioritize the most promising drug-disease pairs candidates (Berrar and Flach, 2012). Additionally, considering that not all drug-disease pairs have been clinically studied. a number of the negative labels might be falsely classified as positive. To address these issues, the methodology was evaluated based on the ratio of true positives that appear in the prioritized drug-disease pairs (i.e., precision) and assessed whether the prioritized drug-disease pairs found through the methodology contained a larger proportion of positive pairs than expected on average by chance. As an additional validation, the results of the methodology were benchmarked against 11 equivalent approaches that can be used to prioritize drug-disease pairs based solely on network structure, as outlined by Abbas *et al.* (2021), Co 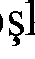 kun and Koyutürk (2021) and Zietz *et al.* (2020).

## Claims

1. A method of identifying a biological pathway that connects a stimulus and a condition in an individual, the method performed at a system comprising one or more processors, the method comprising:
filtering a stimulus-condition knowledge graph using stimulus signature data to obtain a stimulus-filtered knowledge graph,
wherein the stimulus-condition knowledge graph comprises: (a) nodes, each node representing the stimulus, biological entity, or the condition; and (b) causal edges, each causal edge representing a causal relationship connecting two nodes,
wherein the stimulus signature data comprises information pertaining to biological entities associated with the stimulus, and
wherein the stimulus-filtered knowledge graph comprises one or more qualifying paths according to the stimulus signature data;
filtering the stimulus-filtered knowledge graph using condition signature data to obtain a condition-filtered knowledge graph to identify the biological pathway that connects the stimulus and the condition,
wherein the condition signature data comprises information pertaining to biological entities associated with the condition, and
wherein the condition-filtered knowledge graph comprises one or more qualifying paths according to the condition signature data.

2. A method of identifying a biological pathway that connects a stimulus and a condition in an individual, the method performed at a system comprising one or more processors, the method comprising:
filtering a stimulus-condition knowledge graph using condition signature data to obtain a condition-filtered knowledge graph,
wherein the stimulus-condition knowledge graph comprises: (a) nodes, each node representing the stimulus, biological entities, or the condition; and (b) causal edges, each causal edge representing a causal relationship connecting two nodes,
wherein the condition signature data comprises information pertaining to biological entities associated with the condition, and
wherein the condition-filtered knowledge graph comprises one or more qualifying paths according to the condition signature data; and
filtering the condition-filtered knowledge graph using stimulus signature data to obtain a stimulus-filtered knowledge graph to identify the biological pathway that connects the stimulus and the condition,
wherein the stimulus signature data comprises information pertaining to biological entities associated with the stimulus, and
wherein the stimulus-filtered knowledge graph comprises one or more qualifying paths according to the stimulus signature data.

3. The method of claim 1 or 2, further comprising obtaining the stimulus-condition knowledge graph.

4. The method of claim 3, wherein the stimulus-condition knowledge graph is obtained from a master knowledge graph.

5. The method of any one of claims 1-4,
wherein one or more paths connecting the stimulus and the condition of the stimulus-condition knowledge graph each have a length of 10 or fewer causal edges; and/or
wherein at least one of the one or more paths comprise a linear path; and/or
wherein at least one the one or more paths comprise a cyclical path.

6. The method of any one of claims 1-5, wherein the causal relationship represented by the causal edge is based on a stimulus-biological entity interaction, a biological entity-biological entity interaction, or a biological entity-condition interaction.

7. The method of claim 6,
wherein the causal relationship of the stimulus-biological entity interaction represents an activation of the biological entity by the stimulus; or
wherein the causal relationship of the stimulus-biological entity interaction represents an inhibition of the biological entity by the stimulus; or
wherein the causal relationship of the biological entity-biological entity interaction represents an activation of a first biological entity by a second biological entity; or
wherein the causal relationship of the biological entity-condition interaction represents a promotion of a condition by a biological entity.

8. The method of any one of claims 1-7,
wherein the stimulus signature data comprises up-regulation and/or down-regulation information for each biological entity of the stimulus signature data in the presence of the stimulus; and/or
wherein the stimulus signature data comprises information obtained from a database or proprietary source; and/or
wherein the stimulus signature data comprises information obtained from a stimulus-perturbation experiment.

9. The method of any one of claims 1-8, further comprising generating and displaying a visual representation comprising the stimulus-filtered knowledge graph.

10. The method of any one of claims 1-9,
wherein the condition signature data comprises up-regulation and/or down-regulation information for each biological entity of the condition signature data due to the condition; and/or
wherein condition signature data comprises information obtained from a database or proprietary source; and/or
wherein the condition signature data comprises information obtained from a condition-perturbation experiment; and/or
wherein the condition signature data comprises transcriptomic information.

11. The method of any one of claims 1-10,
wherein the one or more qualifying paths comprises a set of causal edges in complete agreement with the stimulus signature data and/ or condition signature data; or
wherein the one or more qualifying paths comprises a set of causal edges comprising one error that is not in agreement with the stimulus signature data and/or condition signature data; or
further comprising scoring paths of the identified biological pathway connecting the stimulus and the condition.

12. The method of any one of claim 1-11, further comprising determining a target biological entity based on the identified biological pathway for further assessment.

13. The method of any one of claims 1-12, wherein the stimulus is a drug or an environmental stimulus.

14. The method of any one of claims 1-13,
wherein the condition is a disease; and/or
wherein the biological entity is a polypeptide.

15. The method of any one of claims 1-14,
wherein the filtering the stimulus-condition knowledge graph is based on concordance; and/or
wherein the filtering the stimulus-filtered knowledge graph is based on concordance; and/or
wherein the filtering the condition-filtered knowledge graph is based on concordance.

## Patentansprüche

1. Verfahren zur Identifizierung einer biologischen Bahn, die einen Stimulus und einen Erkrankungszustand in einem Individuum verbindet, wobei das Verfahren an einem System durchgeführt wird, das einen oder mehrere Prozessoren umfasst, wobei das Verfahren umfasst:
Filtern eines Stimulus-Erkrankungszustand-Wissensgraphen unter Verwendung von Signaturdaten des Stimulus, um einen stimulusgefilterten Wissensgraphen zu erlangen,
wobei der Stimulus-Erkrankungszustand-Wissensgraph umfasst: (a) Knoten, wobei jeder Knoten den Stimulus, eine biologische Entität oder den Erkrankungszustand repräsentiert; und (b) kausale Kanten, wobei jede kausale Kante eine kausale Beziehung repräsentiert, die zwei Knoten verbindet,
wobei die Signaturdaten des Stimulus Informationen umfassen, die sich auf biologische Entitäten beziehen, die mit dem Stimulus assoziiert sind, und
wobei der stimulusgefilterte Wissensgraph eine oder mehrere qualifizierende Pfadverbindungen gemäß den Signaturdaten des Stimulus umfasst;
Filtern des stimulusgefilterten Wissensgraphen unter Verwendung von Signaturdaten des Erkrankungszustands, um einen erkrankungszustandsgefilterten Wissensgraphen zu erlangen, um die biologische Bahn zu identifizieren, die den Stimulus und den Erkrankungszustand verbindet,
wobei die Signaturdaten des Erkrankungszustands Informationen umfassen, die sich auf biologische Entitäten beziehen, die mit dem Erkrankungszustand assoziiert sind, und
wobei der erkrankungszustandsgefilterte Wissensgraph eine oder mehrere qualifizierende Pfadverbindungen gemäß den Signaturdaten des Erkrankungszustands umfasst.

2. Verfahren zur Identifizierung einer biologischen Bahn, die einen Stimulus und einen Erkrankungszustand in einem Individuum verbindet, wobei das Verfahren an einem System durchgeführt wird, das einen oder mehrere Prozessoren umfasst, wobei das Verfahren umfasst:
Filtern eines Stimulus-Erkrankungszustand-Wissensgraphen unter Verwendung von Signaturdaten des Erkrankungszustands, um einen erkrankungszustandsgefilterten Wissensgraphen zu erlangen,
wobei der Stimulus-Erkrankungszustand-Wissensgraph umfasst: (a) Knoten, wobei jeder Knoten den Stimulus, biologische Entitäten oder den Erkrankungszustand repräsentiert; und (b) kausale Kanten, wobei jede kausale Kante eine kausale Beziehung repräsentiert, die zwei Knoten verbindet,
wobei die Signaturdaten des Erkrankungszustands Informationen umfassen, die sich auf biologische Entitäten beziehen, die mit dem Erkrankungszustand assoziiert sind, und
wobei der erkrankungszustandsgefilterte Wissensgraph eine oder mehrere qualifizierende Pfadverbindungen gemäß den Signaturdaten des Erkrankungszustands umfasst; und
Filtern des erkrankungszustandsgefilterten Wissensgraphen unter Verwendung von Signaturdaten des Stimulus, um einen stimulusgefilterten Wissensgraphen zu erlangen, um die biologische Bahn zu identifizieren, die den Stimulus und den Erkrankungszustand verbindet,
wobei die Signaturdaten des Stimulus Informationen umfassen, die sich auf biologische Entitäten beziehen, die mit dem Stimulus assoziiert sind, und
wobei der stimulusgefilterte Wissensgraph eine oder mehrere qualifizierende Pfadverbindungen gemäß den Signaturdaten des Stimulus umfasst.

3. Verfahren nach Anspruch 1 oder 2, ferner umfassend das Erlangen des Stimulus-Erkrankungszustand-Wissensgraphen.

4. Verfahren nach Anspruch 3, wobei der Stimulus-Erkrankungszustand-Wissensgraph von einem Master-Wissensgraphen erlangt wird.

5. Verfahren nach einem der Ansprüche 1-4,
wobei ein oder mehrere Pfade, die den Stimulus und den Erkrankungszustand des Stimulus-Erkrankungszustand-Wissensgraphen verbinden, jeweils eine Länge von 10 oder weniger kausalen Kanten aufweisen; und/oder
wobei mindestens einer des einen oder der mehreren Pfade einen linearen Pfad umfasst; und/oder
wobei mindestens einer des einen oder der mehreren Pfade einen zyklischen Pfad umfasst.

6. Verfahren nach einem der Ansprüche 1-5, wobei die kausale Beziehung, die durch die kausale Kante repräsentiert wird, auf einer Stimulus-biologische Entität-Interaktion, einer biologische Entität-biologische Entität-Interaktion oder einer biologische Entität-Erkrankungszustand-Interaktion basiert.

7. Verfahren nach Anspruch 6,
wobei die kausale Beziehung der Stimulus-biologische Entität-Interaktion eine Aktivierung der biologischen Entität durch den Stimulus repräsentiert; oder
wobei die kausale Beziehung der Stimulus-biologische Entität-Interaktion eine Inhibierung der biologischen Entität durch den Stimulus repräsentiert; oder
wobei die kausale Beziehung der biologische Entität-biologische Entität-Interaktion eine Aktivierung einer ersten biologischen Entität durch eine zweite biologische Entität repräsentiert; oder
wobei die kausale Beziehung der biologische Entität-Erkrankungszustand-Interaktion eine Förderung eines Erkrankungszustands durch eine biologische Entität repräsentiert.

8. Verfahren nach einem der Ansprüche 1-7,
wobei die Signaturdaten des Stimulus Hochregulierungs- und/oder Herunterregulierungsinformationen für jede biologische Entität der Signaturdaten des Stimulus in Gegenwart des Stimulus umfassen; und/oder
wobei die Signaturdaten des Stimulus Informationen umfassen, die von einer Datenbank oder proprietären Quelle erlangt werden; und/oder
wobei die Signaturdaten des Stimulus Informationen umfassen, die von einem Stimulus-Perturbationsexperiment erlangt werden.

9. Verfahren nach einem der Ansprüche 1-8, ferner umfassend das Erzeugen und Anzeigen einer visuellen Darstellung, die den stimulusgefilterten Wissensgraph umfasst.

10. Verfahren nach einem der Ansprüche 1-9,
wobei die Signaturdaten des Erkrankungszustands Hochregulierungs- und/oder Herunterregulierungsinformationen für jede biologische Entität der Signaturdaten des Erkrankungszustands aufgrund des Erkrankungszustands umfassen; und/oder
wobei die Signaturdaten des Erkrankungszustands Informationen umfassen, die von einer Datenbank oder proprietären Quelle erlangt werden; und/oder
wobei die Signaturdaten des Erkrankungszustands Informationen umfassen, die von einem Erkrankungszustand-Perturbationsexperiment erlangt werden; und/oder
wobei die Signaturdaten des Erkrankungszustands transkriptomische Informationen umfassen.

11. Verfahren nach einem der Ansprüche 1-10,
wobei die ein oder mehreren qualifizierenden Pfadverbindungen einen Satz kausaler Kanten in vollständiger Übereinstimmung mit den Signaturdaten des Stimulus und/oder den Signaturdaten des Erkrankungszustands umfassen; oder
wobei die eine oder die mehreren qualifizierenden Pfadverbindungen einen Satz kausaler Kanten umfassen, der einen Fehler umfasst, der nicht in Übereinstimmung mit den Signaturdaten des Stimulus und/oder den Signaturdaten des Erkrankungszustands ist; oder
ferner umfassend die Bewertung von Pfaden der identifizierten biologischen Bahn, die den Stimulus und den Erkrankungszustand verbindet.

12. Verfahren nach einem der Ansprüche 1-11, ferner umfassend das Bestimmen einer biologischen Zielentität basierend auf der identifizierten biologischen Bahn zur weiteren Bewertung.

13. Verfahren nach einem der Ansprüche 1-12, wobei der Stimulus ein Arzneimittel oder ein Umweltstimulus ist.

14. Verfahren nach einem der Ansprüche 1-13,
wobei der Erkrankungszustand eine Krankheit ist; und/oder
wobei die biologische Entität ein Polypeptid ist.

15. Verfahren nach einem der Ansprüche 1-14,
wobei das Filtern des Stimulus-Erkrankungszustand-Wissensgraphen auf Übereinstimmung basiert; und/oder
wobei das Filtern des stimulusgefilterten Wissensgraphen auf Übereinstimmung basiert; und/oder
wobei das Filtern des erkrankungszustandsgefilterten Wissensgraphen auf Übereinstimmung basiert.

## Revendications

1. Procédé d'identification d'une voie biologique reliant un stimulus et une condition chez un individu, le procédé étant mis en œuvre sur un système comprenant un ou plusieurs processeurs, le procédé comprenant :
le filtrage d'un graphe de connaissances stimulus-condition en utilisant des données de signature de stimulus pour obtenir un graphe de connaissances filtré par stimulus,
dans lequel le graphe de connaissances stimulus-condition comprend : (a) des nœuds, chaque nœud représentant le stimulus, une entité biologique ou la condition ; et (b) des arêtes causales, chaque arête causale représentant une relation causale reliant deux nœuds,
dans lequel les données de signature de stimulus comprennent des informations relatives aux entités biologiques associées au stimulus, et
dans lequel le graphe de connaissances filtré par stimulus comprend un ou plusieurs chemins qualifiants en fonction des données de signature de stimulus ;
le filtrage du graphe de connaissances filtré par stimulus en utilisant des données de signature de condition pour obtenir un graphe de connaissances filtré par condition afin d'identifier la voie biologique qui relie le stimulus et la condition,
dans lequel les données de signature de la condition comprennent des informations relatives aux entités biologiques associées au stimulus, et
dans lequel le graphe de connaissances filtré par condition comprend un ou plusieurs chemins qualifiants en fonction des données de signature de stimulus.

2. Procédé d'identification d'une voie biologique reliant un stimulus et une condition chez un individu, le procédé étant mis en œuvre sur un système comprenant un ou plusieurs processeurs, le procédé comprenant :
le filtrage d'un graphe de connaissances stimulus-condition en utilisant des données de signature de condition pour obtenir un graphe de connaissances filtré par condition,
dans lequel le graphe de connaissances stimulus-condition comprend : (a) des nœuds, chaque nœud représentant le stimulus, des entités biologiques ou la condition ; et (b) des arêtes causales, chaque arête causale représentant une relation causale reliant deux nœuds,
dans lequel les données de signature de condition comprennent des informations relatives aux entités biologiques associées au stimulus, et
dans lequel le graphe de connaissances filtré par condition comprend un ou plusieurs chemins qualifiants selon les données de signature de stimulus ; et
le filtrage du graphe de connaissances filtré par condition en utilisant des données de signature de stimulus pour obtenir un graphe de connaissances filtré par stimulus afin d'identifier la voie biologique qui relie le stimulus et la condition,
dans lequel les données de signature de stimulus comprennent des informations relatives aux entités biologiques associées au stimulus, et
dans lequel le graphe de connaissances filtré par stimulus comprend un ou plusieurs chemins qualifiants en fonction des données de signature du stimulus.

3. Procédé selon la revendication 1 ou 2, comprenant en outre l'obtention du graphe de connaissances stimulus-condition.

4. Procédé selon la revendication 3, dans lequel le graphe de connaissances stimulus-condition est obtenu à partir d'un graphe de connaissances maître.

5. Procédé selon l'une quelconque des revendications 1 à 4,
dans lequel un ou plusieurs chemins reliant le stimulus et la condition du graphe de connaissances stimulus-condition ont chacun une longueur de 10 arêtes causales ou moins ; et/ou
dans lequel au moins l'un de l'un ou des plusieurs chemins comprend un chemin linéaire ; et/ou
dans lequel au moins l'un de l'un ou des plusieurs chemins comprend un chemin cyclique.

6. Procédé selon l'une quelconque des revendications 1 à 5, dans lequel la relation causale représentée par l'arête causale est basée sur une interaction stimulus-entité biologique, une interaction entité biologique-entité biologique ou une interaction entité biologique-condition.

7. Procédé selon la revendication 6,
dans lequel la relation causale de l'interaction stimulus-entité biologique représente une activation de l'entité biologique par le stimulus ; ou
dans lequel la relation causale de l'interaction stimulus-entité biologique représente une inhibition de l'entité biologique par le stimulus ; ou
dans lequel la relation causale de l'interaction entité biologique-entité biologique représente une activation d'une première entité biologique par une deuxième entité biologique ; ou
dans lequel la relation causale de l'interaction entité biologique-condition représente une promotion d'une condition par une entité biologique.

8. Procédé selon l'une quelconque des revendications 1 à 7,
dans lequel les données de signature de stimulus comprennent des informations sur la régulation positive et/ou négative pour chaque entité biologique des données de signature de stimulus en présence du stimulus ; et/ou
dans lequel les données de signature de stimulus comprennent des informations obtenues à partir d'une base de données ou d'une source propriétaire ; et/ou
dans lequel les données de signature de stimulus comprennent des informations obtenues à partir d'une expérience de perturbation de stimulus.

9. Procédé selon l'une quelconque des revendications 1 à 8, comprenant en outre la génération et l'affichage d'une représentation visuelle comprenant le graphe de connaissances filtré par stimulus.

10. Procédé selon l'une quelconque des revendications 1 à 9,
dans lequel les données de signature de condition comprennent des informations sur la régulation positive et/ou négative pour chaque entité biologique des données de signature de condition imputées à la condition ; et/ou
dans lequel les données de signature de condition comprennent des informations obtenues à partir d'une base de données ou d'une source propriétaire ; et/ou
dans lequel les données de signature de condition comprennent des informations obtenues à partir d'une expérience de perturbation de condition ; et/ou
dans lequel les données de signature de condition comprennent des informations transcriptomiques.

11. Procédé selon l'une quelconque des revendications 1 à 10, dans lequel l'un ou les plusieurs chemins qualifiants comprennent un ensemble d'arêtes causales en parfait accord avec les données de signature de stimulus et/ou les données de signature de condition ; ou
dans lequel l'un ou les plusieurs chemins qualifiants comprennent un ensemble d'arêtes causales comprenant une erreur qui n'est pas en accord avec les données de signature du stimulus et/ou les données de signature de condition ; ou
comprenant en outre des voies de notation de la voie biologique identifiée reliant le stimulus et la condition.

12. Procédé selon l'une quelconque des revendications 1 à 11, comprenant en outre la détermination d'une entité biologique cible basée sur la voie biologique identifiée pour une évaluation plus approfondie.

13. Procédé selon l'une quelconque des revendications 1 à 12, dans lequel le stimulus est un médicament ou un stimulus environnemental.

14. Procédé selon l'une quelconque des revendications 1 à 13, dans lequel la condition est une maladie ; et/ou dans lequel l'entité biologique est un polypeptide.

15. Procédé selon l'une quelconque des revendications 1 à 14, dans lequel le filtrage du graphe de connaissances stimulus-condition est basé sur la concordance ; et/ou
dans lequel le filtrage du graphe de connaissances stimulus-condition est basé sur la concordance ; et/ou
dans lequel le filtrage du graphe de connaissances filtré par condition est basé sur la concordance.
